# EUROPEAN PATENT SPECIFICATION

(11) **EP 1 292 710 B1**
(45) Date of publication and mention of the grant of the patent: **29.08.2007**
(21) Application number: 01943634.4
(22) Date of filing: 22.06.2001
(51) Int. Cl.: C12Q 1/68

(54) **MATRIX SCREENING METHOD**
MATRIX-SCREENING-VERFAHREN
PROCEDE DE DEPISTAGE PAR UNE MATRICE

(30) Priority: 23.06.2000 GB 0015443; 25.10.2000 GB 0026099; 08.11.2000 US 246851 P
(43) Date of publication of application: 19.03.2003
(73) Proprietor: Domantis Limited, Cambridge Cambridgeshire, CB4 OWG (GB)
(72) Inventor: HOLT, Lucy Jessica, Trumpington, Cambridge CB2 2JE (GB); TOMLINSON, Ian, Great Shelford, Cambridge CB2 5LJ (GB)
(74) Representative: Maschio, Antonio
(86) International application number: PCT/GB2001/002831
(87) International publication number: WO 2001/098534

(56) References cited:
- WO-A-01/00866
- WO-A-93/09668
- WO-A-99/28744
- MASKOS U ET AL: "A STUDY OF OLIGONUCLEOTIDE REASSOCIATION USING LARGE ARRAYS OF OLIGONUCLEOTIDES SYNTHESISED ON A GLASS SUPPORT" NUCLEIC ACIDS RESEARCH, IRL PRESS LTD., OXFORD, GB, vol. 21, no. 20, 1993, pages 4663-4669, XP000999271 ISSN: 0305-1048
- UETZ P ET AL: "A COMPREHENSIVE ANALYSIS OF PROTEIN-PROTEIN INTERACTIONS IN SACCHAROMYCES CEREVISIAE" NATURE, MACMILLAN JOURNALS LTD. LONDON, GB, vol. 403, 10 February 2000 (2000-02-10), pages 623-627, XP000938868 ISSN: 0028-0836
- EMILI A Q ET AL: "LARGE-SCALE FUNCTIONAL ANALYSIS USING PEPTIDE OR PROTEIN ARRAYS" NATURE BIOTECHNOLOGY, NATURE PUB. CO, NEW YORK, NY, US, vol. 18, no. 4, April 2000 (2000-04), pages 393-397, XP000919070 ISSN: 1087-0156
- CHEUNG V G ET AL: "MAKING AND READING MICROARRAYS" NATURE GENETICS, NEW YORK, NY, US, vol. 21, January 1999 (1999-01), pages 15-19, XP000961558 ISSN: 1061-4036
- PANDEY AKHILESH ET AL: "Proteomics to study genes and genomes" NATURE, MACMILLAN JOURNALS LTD. LONDON, GB, vol. 405, no. 6788, 15 June 2000 (2000-06-15), pages 837-846, XP002172041 ISSN: 0028-0836

## Description

The present invention relates to a method which can be used to screen two or more repertoires of molecules against one another and/or to create and screen combinatorial repertoires by combining two or more repertoires. In particular, the invention relates to a method whereby two repertoires of molecules can be screened such that substantially all members of the first repertoire are tested against substantially all members of the second repertoire for functional interactions. Furthermore, the invention relates to the creation and screening of antibody repertoires by combining a repertoire of heavy chains with a repertoire of light chains such that antibodies formed by the substantially all combinations of heavy and light chains can be screened against one or more target ligands.

### Introduction

The mapping and sequencing of different genomes will eventually lead to the cloning of all the proteins expressed by these organisms. In order to create interaction maps of these proteins, two-dimensional screens need to be performed so that the binding of every protein to every other protein can be tested.

Two dimensional screens are also required for a number of other applications. For example, techniques such as mouse immunisation coupled with the production of monoclonal antibodies and *in vitro* selection methods such as phage display have been used to simultaneously generate many different antibodies against many different targets. In order to determine which antibodies bind to which targets these pools need to be deconvoluted, which requires a complex screening procedure.

Furthermore, if small molecule drugs are to be generated against human targets for therapy it would be helpful to determine not only the extent of binding of a given human protein to a putative drug candidate but also the extent (if any) of cross-reaction of the same drug candidate with other human proteins or whether other related drugs are better binders and/or less cross-reactive.

All of these examples call for a technique whereby interactions between members of a first set (or repertoire) of molecules can be rapidly tested against all members of a second set (or repertoire) of molecules. To date, such screens are generally performed by dispensing combinations of reagents into compartmentalised wells or on top of one another in the form of spots on a membrane such that all combinations of reagents to be tested are present in separate wells/spots. Therefore if a repertoire of 100 molecules were to be tested against a different repertoire also consisting of 100 molecules, 10,000 wells/spots would be required to exhaustively cover all combinations of members of the two repertoires. The creation of such discontinuously arranged combinations would require, for a two component interaction, twice as many dispensing 'events' as there are wells or spots, in this case 20,000, in addition to any dispensing events that might be required to facilitate or detect the interactions. As the number of members in each repertoire increases linearly, the number of combinations, and hence dispensing events, increases exponentially. Indeed for a three component interaction, involving, say, a repertoire of only 100 antibody heavy chains, a repertoire of only 100 antibody light - chains and a repertoire of only 100 potential antigens, a million 'dispensing' events would be required.

### Summary of the Invention

We have developed a methodology, which we have called Matrix Screening, which can be used to study all possible interactions between all the members in two repertoires of molecules which removes the need to compartmentalise individual combinations of members of these repertoires.

According to a first aspect of the present invention, there is provided a method for screening a first repertoire of members comprising polypeptides against a second repertoire of members comprising polypeptides to identify those members of the first repertoire which interact with members of the second repertoire, comprising:
(a) arranging the first repertoire in at least one first series of continuous lines wherein each line of said first series comprises a member of said first repertoire and arranging the second repertoire in at least one second series of continuous lines wherein each line of said second series comprises a member of said second repertoire, wherein the first and second repertoires form at least one array, such that substantially all members of the first repertoire are juxtaposed to substantially all members of the second repertoire; and
(b) detecting an interaction between polypeptides of the first and second repertoires, thereby identifying those members of the first repertoire that interact with members of the second repertoire.

The invention, in its broadest form, provides a method for screening two repertoires of molecules against one another. Individual members of the two repertoires are spatially configured to enable the juxtaposition of substantially all combinations of members of both repertoires. It will be understood that reference herein to "substantially all combinations" (or "substantially all members") does not exclude that certain juxtapositions may not occur, either by chance or by design. However, the invention does require that two repertoires of molecules be screened against each other simultaneously, and excludes the screening of a single repertoire with individual member(s) of a second repertoire. Preferably, "substantially all" refers to at least 50%, 55%, 60%, 65%, 70%, 75%, 80%, 85%, 90%, 95%, 96%, 97%, 98%, 99% or 100% of the members of a repertoire.

According to the invention, juxtaposition can be arrived at by, for example, creating a series of lines for each of the two repertoires, which intersect one another. The lines can be straight, substantially parallel lines, or curves, or combinations thereof; the only restriction is that substantially all members of the first repertoire should be able to interact substantially all members of the second repertoire. Examples of complementary configurations include straight parallel lines, disposed at an angle to straight parallel lines; concentric circles or polygons, used together with a star of radial lines. The skilled person will be able to imagine many other systems being used to achieve a similar spatial configuration of the repertoire members according to the invention, all being characterised by the dispensation of some form of continuous line, corresponding to each member of the first repertoire, substantially all of which has the ability to intersect substantially all lines, corresponding to substantially all members of the second repertoire.

According to the invention, there is provided a method wherein members of the both the first repertoire and the second repertoire are arranged in a series of lines, each containing a member of the first or second repertoires such that the lines, corresponding to the first repertoire and those corresponding to the second repertoire are contacted with one another so that substantially all members of the first repertoire are juxtaposed with substantially all members of the second repertoire.

In the context of the present invention, "a" member can mean one single member or at least one member. Advantageously, it refers to one single member. However, in an alternative aspect the invention also provides the use of groups consisting of more than one member of the repertoire in each line, channel or tube. Preferably, such groups consist of 10 of fewer members, advantageously 5 or fewer, but at least 2.

The advantage of using intersecting lines, according to the present invention compared to compartmentalised combinatorial screening in the prior art is that as the size of the individual repertoires grow linearly, so does the number of dispensing steps required to screen all combinations of repertoire members. Thus, whereas screening techniques using wells would require 10,000 dispensing steps to screen a 100 by 100 repertoire, screening according to the present invention requires only 200 dispensing steps, Furthermore, since a single dispensing event is used to spatially array each member of each repertoire, comparison of interactions between individual members of the first repertoire with the members of the second repertoire with which it is juxtaposed will be more accurate. In addition, since the present invention uses intersecting lines rather than spots or intersecting channels rather than wells, less positional accuracy is necessary to ensure that substantially all combinations of possible interactions are tested. Thus, if a two-dimensional screen is performed, and one line corresponding to a member of the first repertoire is offset by, for example, 1mm, since it is arranged at an angle to substantially all the lines from the second repertoire, it will still intersect substantially all of them and therefore substantially all combinations of interactions will still have been successfully tested. If, on the other hand, the spots corresponding to a member of the first repertoire are offset by, for example, 1mm, they may miss the spots corresponding to the members of the second repertoire altogether and therefore many combinations of interactions will not have been tested Therefore, the present invention is not only well suited to automated methods of screening but also to manual methods, where positional accuracy cannot be guaranteed and the number of dispensing events must be limited.

As described above, the lines, can be arranged in a variety of formats and can be arranged on a single support, or a plurality of supports. In the simplest configuration, molecules can be manually drawn out in the form of lines on a single support, for example on a nitrocellulose membrane. These lines can also be applied to suitable supports using robotic techniques, which allow the accuracy and density of arrays to be increased to great advantage in the present invention. In an advantageous aspect of the invention, a multi-support system can be used, wherein arrays of lines are prepared on separate supports which are then juxtaposed in order to assess interaction between, the members of the repertoires.

The present invention can also be applied to higher dimensional arrays, for example, those with 3 dimensions. Thus, three component interactions, such as enzyme, substrate and co-factor can be screened using lines, channels or tubes that are arranged in 3 dimensions. Alternatively, the three components could be antibody heavy chain, antibody light chain and antigen, and repertoires thereof can be screened in three dimensions. The screening of repertoires in 2, 3 or higher dimensions according to the present invention is highly advantageous as it reduces the number of dispensing (or pipetting) events that would be required to perform a comprehensive combinatorial screen. Thus, the screening of two repertoires, of, say, 300 members against one another using conventional techniques in the prior art would require at least 90,000 separate dispensing events and the screening of three repertoires, of, say, 300 members against one another would require at least 2.7 million dispensing events. By contrast, the present invention reduces the number of dispensing events to comprehensively screen the same repertoires to 600 or 900, respectively, a huge saving in terms of time and labour.

A multidimensional array can be created in a number of ways. Advantageously, a third dimension is created by stacking filters or other such membranes and relying on capillary action for transferring molecules, or by forcing molecules through the stack by a means such as electrophoresis or osmosis or by piercing the stack or by the use of permeable filters to create the stack.

Moreover, a third dimension can be created by stacking non-permeable layers which at the intersections of channels (for the first and second repertoires) have holes which (once the layers are stacked) form an additional set of channels in a third dimension along which members of a third repertoire can pass. the third dimension can be created using a block of gel or similar such substance, which can be injected with members of the first, second and third repertoires along the x, y and z faces, respectively, thus creating channels in a three-dimensional space which form the array.

Still further, the matrix of interactions between members of the first, second (and optionally third) repertoires of molecules can be created using a network of intersecting tubes or semi-permeable tubes laid adjacent to one another.

The members of the first, second (and optionally third) repertoires of molecules can be replaced over time with different members from the same repertoires so that a new combination or set of interactions can be screened.

Since the present invention can be used to rapidly screen multicomponent and multi-chain interactions, it can also be applied to the simultaneous creation and screening of combinatorial libraries of molecules, for example, antibody or T cell receptor libraries. Thus instead of generating a large combinatorial library of antibodies by combining the heavy and light chain genes and then separately screening the resulting pairings, the pairings themselves can be generated according to the invention and, optionally screened against one or more target antigens. Thus, say, 1000 heavy chains could be drawn as lines in one dimension, and a 1000 light chains can be drawn as lines in another, such that substantially all the heavy chain lines intersect substantially all the light chain lines, forming at their intersection fully functional and folded antibody molecules, which can then be screened with a Juxtaposed antigen, for example coated on a further support which is brought into contact with the intersecting heavy and light chain lines. According to this embodiment, substantially all combinations of 1000 heavy chains and 1000 light chains will have been screened i.e. a total of 1 million different antibodies, using only 2000 dispensing events, rather than the 1 million that would have to be used according to screening techniques in the prior art. This provides a rapid way for 'naive' screening for specific interactions. Thus, for example, a repertoire of heavy chains and a repertoires of light chains, the members (or any related member) of which have never been in contact or selected against a given target antigen (or a related target antigen thereof) can be screened against the target antigen to identify a specific binding heavy and light chain pairing. .

Thus, in a fifth aspect of the present-inveidou a method is provided for creating a combinatorial library of polypeptides comprising two claims, each member of which library comprises one member of a first repertoire of members comprising a heavy and/or light chain polypeptide and one member of a second repertoire of members comprising a heavy and/or light chain polypeptide, which method comprises the step of arranging the first repertoire of members in a first series of continuous lines, and said second repertoire in a second series of continuous lines, such that substantially all members of the first repertoire are juxtaposed to substantially all members of the second repertoire, thereby generating at the points of juxtaposition, a plurality of combinations of polypeptides comprising two chains, thereby creating a combinatorial library of polypeptides comprising two chains.

Preferably, the combinatorial library is an antibody or T cell receptor library and the two repertoires consist of heavy and light chains (in the case of an antibody library) or alpha and beta chains (in the case of a T cell receptor library).

The combinatorial library so produced is preferably screened for interactions with more than one target molecule. Thus, the target molecule can be provided in the form of a group of target molecules, or a repertoire thereof, and screened in a three-dimensional array as described herein.

Preferably, the method according to the invention can be used such that a three-chain polypeptide library is created (and optionally screened) using first, second and third repertoires of molecules in three dimensions.

The pattern of interactions between the first, second (and optionally third) repertoires can be used to identity positive interactions, negative interactions, specific interactions or cross-reactive interactions, or to construct a phylogenic tree inferring the similarity between members of the first repertoire (using the pattern of interactions with the second and/or, optionally third, repertoires), of the second repertoire (using the pattern of interactions with the first and/or, optionally third, repertoires) and/or of the third repertoire (using the pattern of interactions with members the first and/or second repertoires).

Since many of the interactions that will be screened according to the present invention involve polypeptides that have been derived, directly or indirectly, by expression of nucleic acid sequences, it is highly advantageous that the nucleic acids themselves are arranged in lines, channels or tubes according to the invention and expressed to produce their corresponding polypeptides. In this way, intersecting polypeptides from each of the two repertoires will be expressed together. This can assist their association, particularly when the association of the two repertoire members depends on co-operative folding, for example, as in the case of antibodies. In addition, information regarding the interactions of members of the repertoires will be spatially linked to the genetic information which encodes them. This genetic information can be determined by calculating the co-ordinates of the interaction and isolating the corresponding nucleotide sequence data from any point on its line, channel or tube or by isolating the nucleotide sequence data from the intersection itself.

Accordingly, in a sixth aspect of the present invention, a method is provided whereby one or more of the first, second and, optionally, third repertoires comprise a plurality of nucleic acid molecules which are expressed to produce their corresponding polypeptides *in situ* in the array.

Since the present invention concerns the rapid and efficient screening of two or more repertoires against one another, any currently employed techniques for enhancing or disrupting molecular interactions can be used with the invention. Thus, one repertoire can consist of variants of a free hapten and the other repertoire can consist of selected anti-hapten antibodies. By arranging both repertoires in close proximity to an immobilised version of the target hapten molecule the screen can be used to identify those antibodies that are competed for binding to the immobilised target hapten by binding to certain free hapten variants. In this case, the lack of binding would be considered a positive result. Controls for such an experiment can include a line of water alongside the free haptens and a line of non-hapten binding antibodies alongside the and-hapten antibodies. Alternatively, a single free hapten could be used to disrupt binding of members of a repertoire of anti-hapten antibodies to members of a repertoire of different immobilised hapten variants. Other third molecules might include substances that enhance binding of the repertoire members to one another, which can be used itself in the form of a repertoire according to the invention, In this way, a target molecule could be immobilised on a solid support and intersecting repertoires of binders and binder enhancers could be brought into contact with the target molecule. Those skilled in the art will envisage many different combinations of such molecules and repertoire members.

Accordingly, in a further aspect of the present invention a method is provided for screening a first repertoire of molecules against a second repertoire of molecules to identify members of the first and second repertoires whose interactions with one another are dependant on the presence or absence of a third molecule or set of molecules, comprising.
(a) arranging the first repertoire in at least one first series of continuous lines wherein each line of said first series comprises a member of said first repertoire and arranging the second repertoire in at least one second series of continuous lines wherein each line of said second series comprises a member of said second repertoire, wherein the first and second repertoires form at least one array, such that substantially all members of the first repoertoire are juxtaposed to substantially all members of the second repertoire; and
(b) detecting the interactions between members of the first repertoire and the members of the second repertoire in the presence of different concentrations of the third molecule or set of molecules.

In a further aspect of the invention, there is provided a method for screening a first repertoire of molecules against a second repertoire of molecules to identify those members of the first repertoire which do not interact with those members of the second repertoire, comprising:
(a) arranging the first repertoire in at least one first series of continuous lines wherein each line of said first series comprises a member of said first repertoire and arranging the second repertoire in at least one second series of continuous lines wherein each line of said second series comprises a member of said second repertoire, wherein the first and second repertoires form at least one array, such that substantially all members of the first repertoire are juxtaposed to substantially all members of the second repertoire; and
(b) identifying those members of the first and second repertoires that do not interact with one another.

The method of the present invention bridges the gap between the initial identification of lead targets and molecules from very large repertoires and the final identification of targets or drugs for therapeutic intervention. This problem is addressed in the prior art by use of ELISA screening of possible positive interactants. However, protocols for ELISA are not easily automated for high throughput. The highly parallel nature of the method according to the present invention will reveal comprehensive interaction profiles for members of each repertoire. This will enable, for example, ligands that interact with an entire family of proteins to be distinguished form those which react with only a subset of that family, cross-reactive drugs to be eliminated from development programmes, and the true specificity and cross-reactivity of antibodies to be determined The determination of an antibodies cross-reactivity and hence its specificity is of vital importance where there is a panel of different antibodies have been derived from an immunised mouse or from an *in vitro* selections performed, for example, by phage display. Matrix Screening is particularly powerful in this context as it enables a comprehensive range of antigens to be tested against each antibody in the panel, minimising the chance of unknown and unwanted cross reactivities disrupting downstream investigations.

Alternatively, by using the present invention to create and screen large comprehensively combinatorial libraries, one million clone antibody libraries could be created and screened using only 2,000 dispensing events. In addition, complex protein-protein interaction maps can be created from enriched sources of interacting pairs, or possibly using entire proteomes together with very high density matrices according to the invention.

The invention also incorporates the key advantages of phage display and other expression-display techniques, namely that the nucleic acids encoding the members of a polypeptide repertoire can be spatially associated with their corresponding polypeptides and can thus be selected on the basis of the functional characteristics of the individual polypeptide. Unlike phage display, however, in which this association is achieved by compartmentalising the nucleic acids and the polypeptides using bacterial cells which display the polypeptides on their surfaces, the subject invention advantageously exploits a novel arraying strategy to provide this association. By eliminating the requirement for the nucleic acids and the polypeptides to be retained in or on bacterial cells, the present invention can be extended beyond selection of binding activities to select any polypeptide repertoire on the basis of any functional property of the polypeptides, including enzymatic activity, conformation or any other detectable characteristic.

Further aspects of the present invention are methods provided as follows.

A method for screening two or more repertoires of enzymes which comprise a two or more enzymic reaction to identify those members of the first repertoire and those members of the second repertoire which together create a given product from a given substrate, which method comprises:
(a) arranging the first repertoire in at least one series of continuous lines wherein each line of said first series comprises a member of said first repertoire and arranging the second repertoire in at least one series of second continuous lines wherein each line of said second series comprises a member of said second repertoire, wherein the first and second repertoires form at least one array, such that substantially all members of the first repertoire are juxtaposed to substantially all members of the second repertoire; and
(b) detecting the formation of product at the intersections of the members of the first and the second repertoires.

A method for screening different cellular populations against different viral populations to identify those viral populations that infect those cellular populations, which method comprises:
(a) arranging the cellular populations and the viral populations to form at least one array, wherein the viral populations is arranged in at least one first series of continuous lines wherein each line of said viral population comprises a member of said population and arranging the cellular population in at least one second series of continuous lines wherein each line of said second series comprises a member of said cellular population, wherein the viral and cellular populations form at least one array, such that substantially all members of the cellular population are juxtaposed to substantially all members of the viral population; and
(b) detecting those viral populations that infect those cellular populations.

A method for screening different cellular fractions against one another to identify those cellular fractions that contain components which interact with components in other cellular fractions, which method comprises:
(a) arranging the cellular fractions in at least two series of continuous lines wherein each line of said first and second series comprises a member of said cellular fraction and such that substantially all the different cellular fractions are juxtaposed to one another.
(b) detecting the interaction/s at the intersections of the different cellular fractions.

A method for screening a peptide repertoire against the same peptide repertoire to identify those members of the peptide repertoire that interact with other members of the peptide repertoire, which method comprises:
(a) arranging the members of the peptide repertoire in at least in at least two series of continuous lines wherein each line of said first and second series comprises a member of said peptide repertoire and such that substantially all the different members of the peptide repertoire are juxtaposed to one another
(b) detecting the interaction/s at the intersections of the different members of the peptide repertoire.

49 A method for creating (and optionally screening) a yeast two hybrid library consisting of substantially all members of a first repertoire of polypeptides paired with substantially all members of a second repertoire of polypeptides, which method comprises:
(a) arranging yeast cells containing a plurality of nucleotide sequences to create at least one array, wherein the nucleotide sequences are arranged in at least one first series of continuous lines wherein each line of said first series comprises yeast cells containing a nucleotide sequence and arranging the second repertoire in at least one second series of continuous lines wherein each line of said second series comprises yeast cells containing a member of said second repertoire, wherein the first and second repertoires form at least one array, such that substantially ail members of the first repertoire are juxtaposed to substantially all members of the second repertoire;
(b) allowing the yeast cells containing the members of the first repertoire to mate with the yeast cells containing the members of the second repertoire where the two repertoires intersect; and optionally
(c) expressing the nucleotide sequences to produce the corresponding polypeptides of the first and second repertoires; and optionally
(d) detecting the interaction/s between the polypeptide members of the first and second repertoires.

Various apparatus can be supplied in association with reagents or tools for performing the screens described above.

### Definitions

The term "repertoire" as used according to the present invention refers to a population of diverse variants, for example polypeptide variants which differ in amino acid sequence, DNA variants that differ in nucleotide composition and/or sequence or any other type of molecule which can exist in a number of different forms. Generally, a repertoire includes more than 10 different variants. Large repertoires comprise the highest number of possible variants for selection and can be up to 10¹³ in size. Smaller repertoires are particularly useful, especially if they have been pre-selected to enrich for a particularly useful subset (for example, antibodies that bind cell surface markers, enzymes that catalyse a certain set of reactions, proteins that bind to other proteins etc) or to remove unwanted members (such as those including stop codons, incapable of correct folding or which are otherwise inactive). Such smaller repertories can comprise 10, 10², 10³, 10⁴, 10⁵, 10⁶ or more polypeptides. Advantageously, smaller repertoires comprise between 10 and 10⁴ polypeptides.

In the present invention, two or more repertoires of polypeptides are screened against each other. Advantageously, at lest 50% of the members or each repertoire are screened against each other in each screen. Preferably, 60%, 70%, 80%, 90%, 95% or even 100% of the members of each repertoire are so screened.

In the context of the present invention, "interact" refers to any detectable interaction between the molecules which comprise the various repertoires and, optionally, any additional molecules that comprise the screen. For example, in the case of antibody-antigen interactions one repertoire might comprise a diverse population of antibodies and the other a diverse population of antigens, the interaction being a binding interaction. Alternatively, the interaction can be an enzymatically-catalysed reaction, in which one repertoire is composed of enzymes and the other repertoire is composed of substrates therefor. Any interaction can be assayed using the present invention, including binding interactions, DNA methylation, nucleic acid degradation, nucleic acid cleavage (single or double stranded), Signalling events, catalytic reactions, phosphorylation events, glycosylation events, proteolytic cleavage, chemical reactions, cellular infection and combinations thereof. The detection of such interactions is well known in the art.

In the context of the present invention, "molecule" refers to any substance which can be applied to the screen. Such molecules can include peptides, polypeptides, nucleic acid molecules, purified proteins, recombinant proteins, amino acids, cDNAs, expressed cDNAs, oligonucleotides, nucleotides, nucleotide analogues, families of related genes or the corresponding proteins thereof, enzymes, DNA binding proteins, immunoglobulin family members, antibodies, T cell receptors, haptens, small organic molecules, non-organic compounds, metal ions, carbohydrates and combinations thereof. The creation of repertoires of such molecules is well know in the art. "Polypeptides" can refer to polypeptides such as expressed cDNAs, members of the immunoglobulin superfamily, such as antibody polypeptides or T-cell receptor polypeptides. Advantageously, antibody repertoires can comprise repertoires comprising both heavy chain (V_{H}) and light chain (V_{L}) polypeptides, which are either pre-assembled or assembled and screened according to the present invention. An antibody polypeptide, as used herein, is a polypeptide which either is an antibody or is a part of an antibody, modified or unmodified. Thus, the term antibody polypeptide includes a heavy chain, a light chain, a heavy chain-light chain dimer, a Fab fragment, a F(ab')₂ fragment, a Dab fragment, a light or heavy chain single domain, and an Fv fragment including a single chain Fv (scFv) or a di-sulphide bonded Fv (dsFv). Methods for the construction of such antibody molecules and nucleic acids encoding them are well known in the art However, "polypeptides" can refer to other polypeptides, such as enzymes, antigens, drugs, molecules involved in cell signalling, such as receptor molecules, or one or more individual domains of larger polypeptides, which are capable of an interaction with a target molecule. Molecules according to the invention can be provided in cellular form, that is in the form of cells producing a molecule as described above, or in non-cellular form, that is not contained within cells. Cells can be, for example, bacterial cells, lower eukaryotic cells (e.g., yeasts), or higher eukaryotic cells (e.g., insect, amphibian, avian or mammalian cells).cells. The use of yeast cells is also envisaged.

In the context of the present invention, the term "cellular population" refers to a collection of cells. The cells comprising a cellular population may all be of the same species and cell type, or they may be a mixed population. One embodiment of a cellular population comprises an-essentially substantially uniform population of cells, for example mammalian fibroblasts, transformed with a library encoding variants of a given gene coding sequence.

In the context of the present invention, the term "viral population" refers to a collection of virus particles. The particles comprising a viral population may all be of the same species and strain, or they may be a mixed population. One embodiment of a viral population comprises population of recombinant or randomly mutagenized particles, for example retroviral particles. A viral population can comprise multiple individuals carrying variations of one or more gene coding sequences.

"Juxtaposition", in the context of the present invention, includes but is not limited to physical contact. Two or more repertoires according to the invention can be juxtaposed such that the molecules are capable of interacting with one another in such a manner that the sites of interactions between the members of the repertoires can be correlated with their position Alternatively, the repertoires can be juxtaposed with one another and with a target molecule such that the members of the repertoires interact with one another and then together interact with a target molecule.

An "array" as referred to herein, is a pro-determined spatial arrangement of the members of the repertoire. The array can take any physical form. The array can be created by manual or automated means and preferred arraying technologies are further described below.

A "dispensing event" is a single event whereby a substance is transferred from one discrete location to a second discrete location. A discrete location can be in the form of a well, a tube, a channel, a spot, a line, a rectangle, a sphere, a cube etc. Examples of single dispensing events include:
(i) pipetting a liquid from one tube or well to a second tube or well. In this case pipetting aliquots of the same liquid into multiple tubes or wells would be considered to be multiple dispensing events, as would dispensing two or more different liquids into the same tube or well, or
(ii) transferring liquid from a source well to a membrane by pin transfer to create a spot of that liquid. In this case spotting a second aliquot from the same source well onto a different destination location on the membrane would be considered a separate dispensing event, or .
(iii) transferring liquid from a single source well to create a single continuous line of liquid on a membrane. In this case creating a second separate line, even of the same liquid, would be considered a separate dispensing event or
(iv) dispensing a solution down a tube or channel. In this case, dispensing a different solution down the same tube or channel, or the same solution down a different tube or channel would be considered a separate dispensing event

A "matrix" in the context of the present invention, is a particular kind of array which can be used to study substantially all possible interactions between substantially all the members in two or more repertoires of molecules. Such matrices can comprise a series of intersecting lines, channels or tubes, each containing one or more members of the repertoires. A single matrix will contain many individual lines, channels or tubes and many more intersections, or nodes.

The term "enhanced" as used herein means that a detected interaction is increased by at least 10% in the presence of a given molecule or molecules relative to the interaction in the absence of that molecule or molecules.

The term "blocked" as used herein means that a detected interaction is decreased by at least 10% in the presence of a given molecule or molecules relative to the interaction in the absence of that molecule or molecules.

The term "cellular fraction" as used herein means a portion of a cell lysate resulting from a cell fractionation process. Non-limiting examples of cell fractionation processes include, detergent extraction, salt extraction, acid precipitation, extraction of lipid soluble components, membrane isolation, extraction of water soluble or aqueous components, nucleo/cytoplasmic fractionation, and separations based on centrifugal forces (e.g:, the S-100 fraction). Other separations considered to be cell fractionation processes include nucleic acid isolation, chromatographic separation of components of cell lysate or fractionated cell lysate, preparative electrophoretic fractionation, ion exchange and affinity separations (e.g., immunoprecipitation or immunoaffinity chromatography, His/Ni++ interactions, GST/glutathione interactions, etc.).

### Brief Description of the Drawings

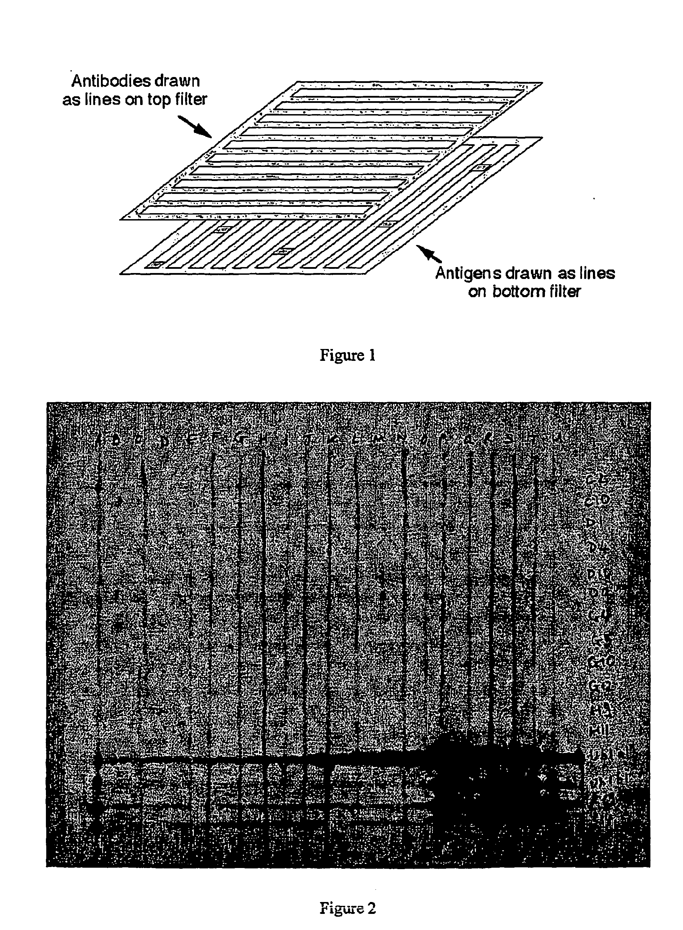

**Figure. 1:** Outline of one method for screening a repertoire of antibodies against a repertoire of antigens according to the present invention, demonstrating how hundreds of different antibodies could be screened simultaneously against hundreds of different antigens to identify interacting pairs. Specific interactions are indicated.
**Figure 2:** Analysis of scFvs using a manually created matrix. Here, 21 antigens (horizontally) are screened against 16 scFvs (vertically). Four scFvs have been selected against ubiquitin by phage selection (Ub1b1, Ub1a1, R13 and R14). Two antigen clones are known to be ubiquitin (Q and T) and five other clones (A, P, R, S and U) have been identified in a primary screen as probably binding an anti-ubiquitin scFv. Each of the four anti ubiquitin scFvs binds the two known ubiquitin clones and each of the five potential ubiquitin clones. However, it can be seen that scFv Ub1b1 and scFv R14 are highly cross reactive.

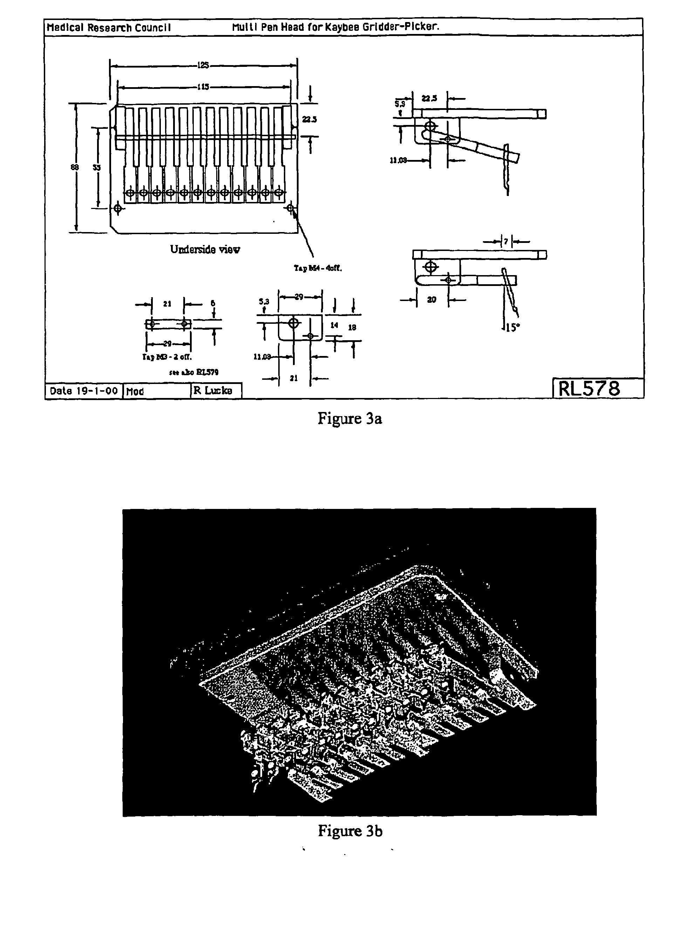

**Figure 3:** A head for robotic line drawing according to the present invention designed for mounting on a robotic platform which allows movement in x, y and z dimensions. A row of fountain pen nibs delivers repertoire members in a liquid suspension, by capillary action to a suitable solid support. The nibs are mounted in such a way as to deliver liquid at an optimum angle to the solid support and then to be held vertical by a stop for loading with liquid from a 96 well microtitre plate.

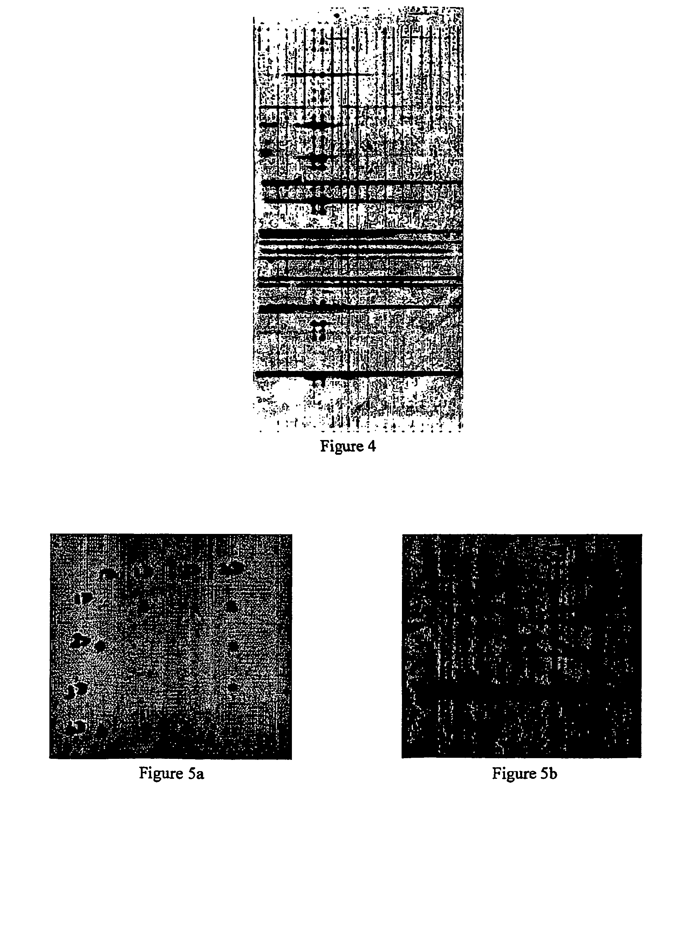

**Figure 4:** Analysis of scFvs using a robotically created matrix. Double lines of 12 antigens (horizontally) are screened against 192 scFvs (vertically). Specific interactions can be observed at the intersections of specific pairings. In addition, scFvs that cross-react with the nitrocellulose can be seen as continuous horizontal lines as can scFvs that cross-react with substantially all antigens (horizontal spotting).
**Figure 5:** Example of creation and screening of a two-chain antibody repertoire, (a) A spotted array according to the prior art. Bacteria that secrete 1. Bovine Serum Albumin (BSA) binding heavy chains, 2. BSA binding light chains, 3. non binding heavy and light chains or 4. BSA binding heavy and light chains were mixed and then grown and induced in close proximity to immobilised BSA indicating that 1 and 2 need to be mixed to get a binding antibody (as seen in the control, 4). 32 separate dispensing events were required to produce this screen. (b) A matrix screen according to the present invention allows the same screen to be performed using only 8 dispensing events (the lack of a signal for 1 down with 2 across is probably due to a bubble being present between the filters during induction).

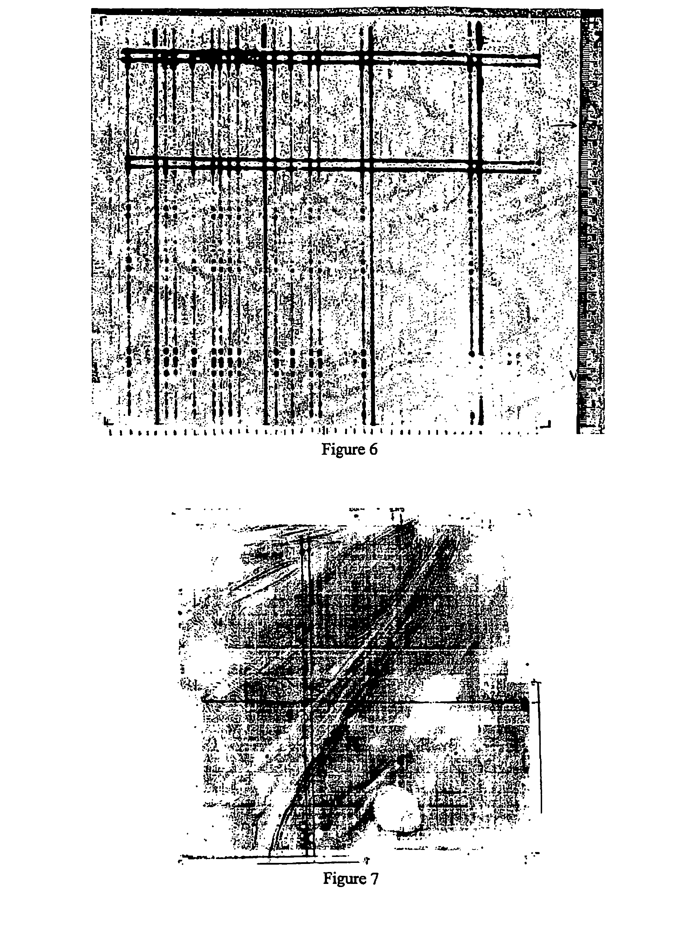

**Figure 6:** By increasing the density of the heavy and light chain lines higher density antibody arrays can be created and screened. Thus, 24 anti-BSA heavy chains and 48 anti-BSA light chains were drawn perpendicular to the x and y axes to create 1152 pairings screened against BSA.

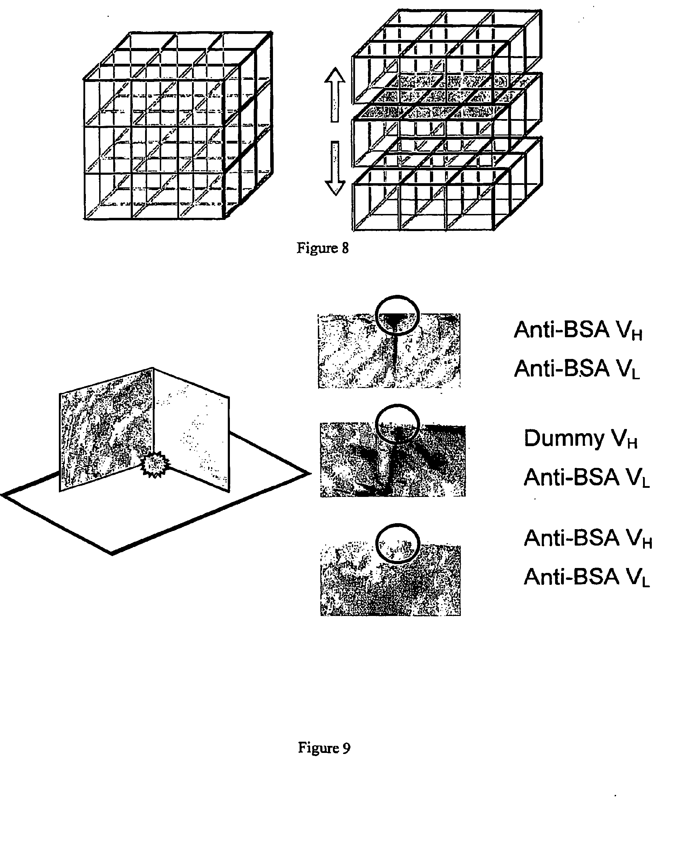

**Figure 7:** 384 unselected heavy chains and 384 unselected light chains were drawn perpendicular to the x and y axes and screened against BSA coated onto a nitrocellulose filter (147,456 combinations). A single specific heavy and light chain pairing was isolated which was subsequently confirmed as binding to nitrocellulose.
**Figure 8**: Schematic for three-dimensional screening according to the invention. Here, members of the repertoires are arranged in planes in the x, y and z axes and the interactions occur at the various vertices of the intersecting planes.
**Figure 9:** Proof of concept of a three-dimensional screen. The anti-BSA heavy chain is deposited on one plane, the anti-BSA light chain is deposited on a second plane, and BSA is deposited in the third plane. An interaction at their vertex is detected only when all three are present.

### Detailed Description of the Invention .

Matrices according to the present invention can be generated in many different ways to screen many different interactions involving many different molecules. The invention is characterised by the ability to screen substantially all combinations of members of two or more repertoires. We have shown that this can be performed using a series of intersecting lines but other approaches which allow combinatorial screening of two or more repertoires using a minimum number of dispensing events are envisaged, such as the use of intersecting channels or tubes. Our method relies on the juxtaposition of continuous groupings of molecules to create a web in two or three dimensions whereby members of the different repertoires come together and potentially interact with one another. This contrasts with screening protocols in the prior art, whereby discontinuous spotting or compartmentalised wells are used to segregate individual combinations of molecules. In the present invention, continuous lines, channels or tubes intersect one another such that individual combinations of molecules exist at their points of intersection, or nodes. Taken as a whole, the molecular 'web' or 'network' thereby created can be used not only to identify specific interacting pairs, but also the overall pattern of interactions between two repertoires. The information so provided can be used to compare the performance of members of either of the repertoires with one another and in particular can be used to rapidly identify cross-reactivities of individual repertoire members within the matrix.

### Repertoires for screening

Many different repertoires can be used with the present invention, the construction of which is well known by those skilled in the art Repertoires of small organic molecules can be constructed by methods of combinatorial chemistry. Repertoires of peptides can be synthesised on a set of pins or rods, such as described in WO84/03564. A similar method involving peptide synthesis on beads, which forms a peptide repertoire in which each bead is an individual repertoire member, is described in U.S. Patent No. 4,631,211 and a related method is described in WO92/00091. A significant improvement of the bead-based methods involves tagging each bead with a unique identifier tag, such as an oligonucleotide, so as to facilitate identification of the amino acid sequence of each library member. These improved bead-based methods are described in WO93/06121. Although these repertoires could be constructed prior to arraying to produce the matrix, it is envisaged that all the techniques described above could be adapted for *in situ* synthesis of the repertoire members directly on the matrix itself - thus linking repertoire construction and repertoire screening according to the present invention. Indeed, another chemical synthesis method involves the synthesis of arrays of peptides (or peptidomimetics) on a surface in a manner that places each distinct library member (e.g., unique peptide sequence) at a discrete, predefined location in the array. The identity of each library member is therefore determined by its spatial location in the array. The locations in the array where binding interactions between a predetermined molecule (e.g., a receptor) and reactive library members occur is determined, thereby identifying the sequences of the reactive library members on the basis of spatial location. These methods are described in U.S. Patent No. 5,143,854; WO90/15070 and WO92/10092; Fodor et al. (1991) Science, 251: 767; Dower and Fodor (1991) Ann. Rep. Med Chem, 26: 271 and could be easily be adapted for creation of matrices according to the present invention.

The present invention is especially useful for the screening of protein-protein interactions, particularly antibody-antigen interactions. The preparation of appropriate antibody repertoires useful in the present invention is described in WO 99/20749, the disclosure of which is incorporated herein by reference. WO 99/20749 describes how a library of immunoglobulins can be prepared and preselected using a generic ligand, and/or prepared using a single main-chain conformation. Libraries as described in WO 99/20749 can be expressed in host organisms, as described therein or according to techniques well known in the art, to produce repertoires of polypeptides which are suitable for arraying and use in the present invention. Alternatively, polypeptides can be synthesised *in situ* for use in the present invention, or expressed using *in vitro* transcription/translation.

### Arraying members of the repertoires to create the matrix screen

According to the present invention, molecules can be arrayed by any one of a variety of methods, manual or automated, in order to create a matrix, depending upon whether the molecules are arrayed as such or expressed by arrayed nucleotide precursors, which may or may not be present in host cells. Arrays are advantageously created by robotic means, since robotic techniques allow the creation of precise and condensed matrices, which can be easily replicated so that, for example, a combinatorial antibody repertoire created according to the invention can be screened against many different target ligands. Robotic platforms are well-known in the art, and machines are available from companies such as Genetix, Genetic MicroSystems and BioRobotics which are capable of arraying at high speed with great accuracy over small or large surfaces. Such machines are capable of spotting purified protein, supernatant or cells onto porous or non-pomus surfaces, such that they can subsequently be fixed thereto if necessary to produce stable arrays. Although robotic manipulation is the preferred method for creating high density arrays, any technique, including manual techniques, which is suitable for locating molecules or cells at predefined locations on a support, can be used. Arraying can be regular, such that lines are 'drawn' at a given distance from the next, irregular or random.

The repertoires of molecules can be screened in solution for interactions or one or more of the repertoires can be immobilised on a solid support. Thus, two solutions can flow down two channels such that at their point of intersection an interaction occurs which can be detected by, for example, a colorimetric, fluorescent, or luminescent reaction. Alternatively, one of the repertoires could be immobilised on a nitrocellulose membrane by, for example, cross-linking and then solutions corresponding to a second repertoire could be 'drawn' onto the support harbouring the immobilised members of the first repertoire. Such immobilisation can be direct or indirect For example, indirect immobilisation can involve arraying a polypeptide repertoire onto a solid support coated with a generic ligand.

In one aspect, members of the repertoires are directed to their positions by means of a tagging system, such that each line, binds or is predisposed to bind a particular member of the repertoire. For example, each polypeptide in one member of a repertoire can comprise a tag, such as an epitope for a known antibody, or a member of an affinity pair (e.g., avidin/biotin, etc.). The line, is coated with a corresponding molecule that binds the tag (e.g., an antibody specific for the epitope tag, or the corresponding member of the binding pair). Contacting the coated line, with a solution comprising the tagged member of the repertoire will effect the arrangement of that member on the array.

Alternatively, both repertoires could be immobilised on a separate solid supports and then juxtaposed to identify interacting pairs. In a preferred aspect of the invention, matrices of polypeptides can be created by first arraying their nucleic acid precursors in host cells and then by expressing the nucleotide sequences to produce the corresponding polypeptides.

In one aspect, yeast cells can be used to express one or more repertoires of molecules useful in a method according to the invention. Methods of introducing and expressing exogenous nucleic acids in yeast are well known in the art. One preferred approach using yeast takes advantage of yeast two-hybrid techniques. In this approach, one population of yeast is transformed with a library encoding a repertoire of fusions with one member of a two-hybrid pair, and another population is transformed with a library encoding a repertoire of fusions with the corresponding second member of a two-hybrid pair. The two yeast cell populations are of different mating types. The two populations are arranged so as to create an array, such that yeast cells containing all members of the first repertoire intersect with yeast cells containing all members of the second repertoire, and the cells are allowed to mate. Interactions between members of the first repertoire and the second repertoire will generate a signal from an appropriate two-hybrid reporter construct.

In another aspect, insect, amphibian, avian, mammalian or other higher eukaryotic cells can be used. As a non-limiting example, a repertoire of molecules (small organic molecules, peptides, polypeptides, etc.) can be screened for those that interact with a repertoire of modified-recombinant cell surface receptors (e.g., a receptor with a variable cassette inserted in a region instrumental in ligand binding or activation) by creating an array in which each member of the repertoire of molecules intersects with each member of the repertoire of receptors. Subsequent detection of receptor activation or inhibition in the cells indicates which of the molecules affected the activity of which modified receptor. The process permits both the identification of new modulators of the receptor or other protein, and the rapid identification of structure/function relationships. The method can also be adapted to use higher eukaryotic cells for the expression of both repertoires being analysed for interaction. This would be accomplished, for example, by expressing both repertoires as cell surface molecules, or for example, by expressing one repertoire as a secreted protein and the other as a cell surface protein. Upon contact or close juxtaposition of the cells expressing the respective repertoires, productive interaction of members of each repertoire with members of the other can be detected. One skilled in the art can readily generate higher eukaryotic cells expressing a given repertoire of polypeptides.

Methods of detecting interactions will vary with the exact nature of the array generated. For example, methods will vary depending on whether the array uses cells or not Non-limiting examples of detection methods include: fluorescence resonance energy transfer (FRET); fluorescence quenching; reporter expression (e.g., luciferase, GST, chloramphenicol acetyl transferase, β-galactosidase, antibiotic resistance); rescue from auxotrophy; signalling events, such as changes in second messenger levels, GDP for GTP exchange, kinase activation or phosphorylation, phosphatase activation or dephosphorylation, proteolysis or altered ion permeability; enzymatic reactions; methylation; nucleic acid cleavage; glycosylation; proteolysis; and infection (e.g., with a virus or phage). Each of these approaches or read-outs for the detection of interactions is known in the art such that one of ordinary skill can employ them in the methods of the invention without the need for undue experimentation.

### Use of molecules selected according to the invention

Molecules selected according to the method of the present invention can be employed in substantially any process. Where the molecules are polypeptides, they can be used in any process which involve binding or catalysis, including *in* vivo therapeutic and prophylactic applications, *in vitro* and *in vivo* diagnostic applications, *in vitro* assay and reagent applications, and the like. For example, antibody molecules can be used in antibody based assay techniques, such as ELISA, techniques, Western blotting, immunohistochemistry, affinity chromatography and the like, according to methods known to those skilled in the art.

As alluded to above, the molecules selected according to the invention are of use in diagnostic, prophylactic and therapeutic procedures. For example, enzyme, variants generated and selected by these methods can be assayed for activity, either *in vitro* or *in vivo* using techniques well known in the art, by which they are incubated with candidate substrate molecules and the conversion of substrate to product is analysed. Selected cell-surface receptors or adhesion molecules might be expressed in cultured cells which are then tested for their ability to respond to biochemical stimuli or for their affinity with other cell types that express cell-surface molecules to which the undiversified adhesion molecule would be expected to bind, respectively.

Therapeutic and prophylactic uses of proteins prepared according to the invention involve the administration of polypeptides selected according to the invention to a recipient mammal, such as a human. Of particular use in this regard are antibodies, other receptors (including, but not limited to T-cell receptors) or binding protein thereof.

Substantially pure molecules of at least 90 to 95% homogeneity are preferred for administration to a mammal, and 98 to 99% or more homogeneity is most preferred for pharmaceutical uses, especially when the mammal is a human. Once purified, partially or to homogeneity as desired, the selected polypeptides can be used diagnostically or therapeutically (including extracorporeally) or in developing and performing assay procedures, immunofluorescent staining and the like (Lefkovite and Pernis, (1979 and 1981) Immunological Methods, Volumes I and II, Academic Press, NY).

The selected antibodies or binding proteins thereof of the present invention will typically find use in preventing, suppressing or treating inflammatory states, allergic hypersensitivity, cancer, bacterial or viral infection, and autoimmune disorders (which include, but are not limited to, Type I diabetes, multiple sclerosis, rheumatoid arthritis, systemic lupus erythematosus, Crohn's disease and myasthenia gravis).

In the instant application, the term "prevention" involves administration of the protective composition prior to the induction of the disease. "Suppression" refers to administration of the composition after an inductive event, but prior to the clinical appearance of the disease. "Treatment" involves administration of the protective composition after disease symptoms become manifest.

Animal model systems which can be used to screen the effectiveness of the antibodies or binding proteins thereof in protecting against or treating the disease are available. Methods for the testing of systemic lupus erythematosus (SLE) in susceptible mice are known in the art (Knight et al. (1978) J. Exp. Med., 147: 1653; Reinersten et al. (1978) New Eng. J. Med, 299: 515). Myasthenia Gravis (MG) is tested in SJL/J female mice by inducing the disease with soluble AchR protein from another species (Lindstrom et al, (1988) Adv. Immunol., 42: 233). Arthritis is induced in a susceptible strain of mice by injection of Type II collagen (Stuart et al. (1984) Ann. Rev. Immunol., 42: 233). A model by which adjuvant arthritis is induced in susceptible rats by infection of mycobacterial heat shock protein has been described (Van Eden et al. (1988) Nature, 331: 171). Thyroiditis is induced in mice by administration of thyroglobulin as described (Maron et al. (1980) J. Exp. Med., 152: 1115). Insulin dependent diabetes mellitus (IDDM) occurs naturally or can be induced in certain strains of mice such as those described by Kanasawa et al. (1984) Diabetologia, 27: 113. EAE in mouse and rat serves as a model for MS in human. In this model, the demyelinating disease is induced by administration of myelin basic protein (see Paterson (1986) Textbook of Immunopathology, Mischer et al., eds., Grune and Stratton, New York, pp. 179-213; McFarlin et al. (1973) Sience, 179: 478: and Satoh et al. (1987) J. Immunol., 138: 179).

The selected antibodies, receptors (including, but not limited to T-cell receptors) or binding proteins thereof of the present invention can also be used in combination with other antibodies, particularly monoclonal antibodies (MAbs) reactive with other markers on human cells responsible for the diseases. For example, suitable T-cell makers can include those grouped into the so-called "Clusters of Differentation," as named by the First International Leukocyte Differentiation Workshop (Bernhard et al. (1984) Leukocyte Typing, Springer Verlag, NY).

Generally, the present selected antibodies, receptors or binding proteins will be utilised in purified form together with pharmacologically appropriate carriers. Typically, these carriers include aqueous or alcoholic/aqueous solutions, emulsions or suspensions, any including saline and/or buffered media. Parenteral vehicles include sodium chloride solution, Ringer's dextrose, dextrose and sodium chloride and lactated Ringer's. Suitable physiologically-acceptable adjuvants, if necessary to keep a polypeptide complex in - suspension, can be chosen from thickeners such as carboxymethylcellulose, polyvinylpyrrolidone, gelatin and alginates.

Intravenous vehicles include fluid and nutrient replenishers and electrolyte replenishers, such as those based on Ringer's dextrose. Preservatives and other additives, such as antimicrobials, antioxidants, chelating agents and inert gases, can also be present (Mack (1982) Remington's Pharmaceutical Sciences, 16th Edition).

The selected polypeptides of the present invention can be used as separately administered compositions or in conjunction with other agents. These can include various immunotherapeutic drugs, such as cylcosporine, methotrexate, adriamycin or cisplatinum, and immunotoxins. Pharmaceutical compositions can include "cocktails" of various cytotoxic or other agents in conjunction with the selected antibodies, receptors or binding proteins thereof of the present invention, or even combinations of selected polypeptides according to the present invention having different specificities, such as polypeptides selected using different target ligands, whether or not they are pooled prior to administration.

The route of administration pharmaceutical compositions according to the invention of pharmaceutical compositions according to the invention can be any of those commonly known to those of ordinary skill in the art. For therapy, including without limitation immunotherapy, the selected antibodies, receptors or binding proteins thereof of the invention can be administered to any patient in accordance with standard techniques. The administration can be by any appropriate mode, including parenterally, intravenously, intramuscularly, intraperitoneally, transdermally, *via* the pulmonary route, or also, appropriately, by direct infusion with a catheter. The dosage and frequency of administration will depend on the age, sex and condition of the patient, concurrent administration of other drugs, counterindications and other parameter to be taken into account by the clinician.

The selected polypeptides of this invention can be lyophilised for storage and reconstituted in a suitable carrier prior to use. This technique has been shown to be effective with conventional immunoglobulins and art-known lyophilisation and reconstitution techniques can be employed. It will be appreciated by those skilled in the art that lyophilisation and reconstitution can lead to varying degrees of antibody activity loss (e.g. with conventional immunoglobulins, IgM antibodies tend to have greater activity loss than IgG antibodies) and that use levels may have to be adjusted upward to compensate.

The compositions containing the present selected polypeptides or a cocktail thereof can be administered for prophylactic and/or therapeutic treatments. In certain therapeutic applications, an adequate amount to accomplish at least partial inhibition, suppression, modulation, killing, or some other measurable parameter, of a population of selected cells is defined as a "therapeutically-effective dose". Amounts needed to achieve this dosage will depend upon the severity of the disease and the general state of the patient's own immune system, but generally range from 0.005 to 5.0 mg of selected antibody, receptor (e.g. a T-cell receptor) or binding protein thereof *per* kilogram of body weight, with doses of 0.05 to 2.0 mg/kg/dose being more commonly used. For prophylactic applications, compositions containing the present selected polypeptides or cocktails thereof can also be administered in similar or slightly lower dosages.

A composition containing a selected polypeptide according to the present invention can be utilised in prophylactic and therapeutic settings to aid in the alteration, inactivation, killing or removal of a select target cell population in a mammal. In addition, the selected repertoires of polypeptides described herein can be used extracorporeally or *in vitro* selectively to kill, deplete or otherwise effectively remove a target cell population from a heterogeneous collection of cells. Blood from a mammal can be combined extracorporeally with the selected antibodies, cell-surface receptors or binding proteins thereof whereby the undesired cells are killed or otherwise removed from the blood for return to the mammal in accordance with standard techniques,

The invention is further described, for the purpose of illustration only, in the following examples.

### Example 1

### Matrix Screening of a scFv Repertoire

The two filter capture system used as part of the present example is based upon that described in our co-pending UK patent application entitled "Array Screening Method", (UK Patent Application Number: 9928787.2). Bacteria are grown in lines on one filter and the scFvs thereby produced are captured on a second filter that has lines of antigen bound to the nitrocellulose, which are oriented at 90° from those lines of scFv on the first filter (see Fig 1). At intersections where scFv interacts with antigen, the scFv is captured if the antigen and scFv bind. In this example, detection of bound scFv is performed with superantigen Protein L conjugated to HRP.

### Methods

### Antigen library

The antigens are from human expression library hEXl, prepared from foetal brain poly(A)+ RNA by oligo(dT)-priming (Büssow et al 1998). cDNAs are cloned directionally in a modified pQE-30 vector (Qiagen).

### Antigen filters

Antigen clones were grown overnight in liquid culture (2xTY containing 100 µg/ml Amp, and 1 % glucose) at 37°C. For manual line drawing, liquid cultures were transferred to a pre-wetted PVDF filter (soak in ethanol, rinse in PBS and dip in 2xTY) by drawing along the filter against a metal ruler with a p10 filter tip (Art) not mounted on a pipette. Thus, the capillary action of the tip was used for delivery of the liquid onto the surface of the membrane. Each clone was drawn onto the filter 6 mm from the previous one. For automated line drawing, liquid cultures were transferred to a PVDP filter using the robotic head depicted in Fig 3 attached to a Kaybee Systems picker/gridder system. Each clone was drawn onto the filter 4.5mm mm from the previous one at a speed of 25 mm/s.

The antigen filters were then grown overnight on TYE agar plates containing 100 µg/ml Amp, 1 % glucose at 30 °C. The filter was then transferred to another TYE agar plate containing 100 µg/ml Amp, 1mM IPTG for 3h at 37 °C for induction of the clones. Antigen filters were removed from the plate and denatured on pre-soaked blotting paper containing 0.5M NaOH, 1.5 M NaCl for 10 min, neutralised for 2 x 5 min in 1M Tris-HCl, pH7.5, 1.5M NaCl and incubated for 15 min in 2 x SSC. Filters were dried, soaked briefly in ethanol and then blocked in 4 % Marvel PBS, rinsed in PBS and dipped in 2xTY.

### ScFv library

The scFvs are from a library based on a single human framework for V_{H} (V3-23/DP-47 and J_{H}4b) and V_{K} (012/02/DPK9 and Jₖ1), with side chain diversity (NNK or DVT encoded) incorporated at positions in the antigen binding site that make contacts to antigen in known structures and are highly diverse in the mature repertoire. The fold that is used is frequently expressed *in vivo,* and binds to the generic ligands Protein L and A, which facilitate capture or detection of the scFvs but do not interfere with antigen binding. The scFv clones have been pre-screened in scFv format for binding to Protein A and Protein L to ensure that they were functional.

### ScFv filter

Antibody clones were grown overnight in liquid culture (2xTY containing 100 µg/ml ampicillin and 1% glucose) at 37°C. Liquid cultures were then transferred to a pre-blocked nitrocellulose filter (4% skimmed milk powder in PBS for 1 hour at room temperature (RT), rinse in PBS and dip in 2xTY). Manual and robotic transfer of antibodies to the filter was performed as for the antigen cultures, except that the density of scFvs lines created by robotic transfer was one every 1.125 mm.

ScFv filters were then grown on TYE agar plates containing 100 µg/ml Amp, 1 % glucose at 37°C. After overnight growth the antigen filter was placed onto a fresh TYE agar plate 100 µg/ml Amp, 1mM IPTG, dried, and then the scFv filter was placed on top of this. The plate with the two filters was then incubated for 3h at 30°C for induction of the scFvs.

### Probing offilters

The top (scFv) filter was discarded and the second (antigen) filter was washed 3 x with PBS/0.05% Tween (PBST) and blocked with 4% MPBS for 30 min at RT. The filters were washed 3x with PBST and then incubated with a Protein L HRP conjugate (Actigen, 1/2000) in 4% MPBS for 1 hr at RT. Filters were then washed a further three times with PBST and developed with ECL reagent. All incubations were performed in 50 ml of buffer on a gently agitating shaker.

### Results

As a model system, we performed a manual matrix screen of 21 antigens against 16 scFvs, resulting in 336 interactions being tested using only 37 dispensing events. Included in the scFv repertoire were four scFvs that had been selected against ubiquitin by phage selection (Ub1b1, Ub1a1, R13 and R14). Included in the antigen repertoire were two clones known to be ubiquitin (Q and T) sand five other clones (A, P, R, S and U) that had been identified in a primary screen as probably binding an anti ubiquitin scFv. As can be seen (Fig 2), each of the four anti ubiquitin scFvs bound the two known ubiquitin clones and each of the five potential ubiquitin clones. However, it can be seen that scFv Ub1b1 and scFv R14 are highly cross reactive. Also included in the model array were 14 antigen clones identified in a primary antigen array screen as possible binders to twelve scFv clones C2 to H11. As can be seen from the matrix .(Fig 2), antigen M (a protein of unknown function) binds specifically to scFv D12. Also antigen E, (a DNA binding protein) binds specifically to scFv H11. This demonstrates the utility of the matrix screen in confirming interactions originally identified in an antigen array screen.

We then moved to a higher density matrix screen, using a robotic head (Fig 3a - design. Fig 3b - photograph) to draw the lines. In this system double lines of 12 antigens (horizontally) are screened against 192 scFvs (vertically). Thus, 2304 different potential interactions were tested each twice over using only 216 dispensing events. Again many different interactions are observed at the intersections of the lines, particularly against three antigens (two of which are the same).

### Example 2

### Creation and screening of a two-chain antibody repertoire according to the present invention

The two filter capture system used as part of the present example is based upon that described in our co-pending UK patent application entitled "Array Screening Method", (UK patent Application Number: 9928787.2). Previously, it has been shown that antibody heavy and light chains can associate in solution to form Fv fragments that have an active antigen-binding site and such techniques are well known in the art. In order to check whether the non-covalent association of the particular heavy and light chain was of sufficient strength for such association to occur on an array, we split the heavy and light chain of a phage selected anti-BSA scFv (13cg2). As we were unsure how strong the association between heavy and light chains would be, we cloned the 13cg2 heavy and light chains separately into three recombinant fragment formats; heavy or light chain alone (single domains); scFv (with a 15 amino acid linker between heavy and light chain) and diabody (with a zero amino acid linker between heavy and light chain). The latter two formats were constructed with either light or heavy chain 13cg2 diversity, with the non-diversified chain in each case being a dummy heavy or light chain. (The dummy chain has a single but unknown antigen-binding specificity.) Testing of the various formats on the array, using BSA as the antigen, indicates that the diabody formats provide the most stable association on the filter surface.

Bacteria expressing either a Bovine Serum Albumin (BSA) binding heavy chain (I), a BSA binding light chain (2), non binding heavy and light chains (3) or BSA binding heavy and light chains (4), all in the diabody format described above, were either mixed and grown as spots (Figure 5a) or drawn as horizontal and vertical lines and then grown (Figure 5b). In both cases, after overnight growth the filters harbouring the grown bacteria were laid on top of a second filter coated with BSA and then induced for protein expression. Only in those cases where a binding heavy chain is co-expressed with a binding light chain is a positive signal observed (i.e. 1 and 2 together or any combination involving 4. The lack of a signal for 1 down with 2 across is probably due to a bubble being present between the filters during induction). The drawing of lines dramatically reduces the number of dispensing events (in this case from 32 to 8).

By increasing the density of the heavy and light chain lines higher density antibody arrays can be created and screened. Thus, 24 anti-BSA heavy chains and 48 anti-BSA light chains were drawn perpendicular to the x and y axes to create 1152 pairings screened against BSA (Figure 6). In an even higher density format 384 unselected heavy chains and 384 unselected light chains were drawn perpendicular to the x and y axes and screened against BSA coated onto a nitrocellulose filter (147,456 combinations). A single specific heavy and light chain pairing was isolated which was subsequently confirmed as binding to nitrocellulose (Figure 7). If the screen were to be increased to cover 1000 heavy chains versus 1000 light chains (1 million different antibodies) the number of dispensing events would be reduced from 2 million to 2 thousand by using the method according to the present invention.

### Example 3

### Three-dimensional screening

A schematic for three-dimensional screening according to the invention is shown (Figure 8). Here, members of the repertoires are arranged in planes in the x, y and z axes and the interactions occur at the various vertices of the intersecting planes. As a proof of concept, an anti-BSA heavy chain is deposited on one plane, an anti-BSA light chain is deposited on a second plane, and BSA is deposited in the third plane, An interaction at their vertex is detected only when all three are present (Figure 9).

Various modifications and variations of the described methods and system of the invention will be apparent to those skilled in the art without departing from the scope of the invention Although the invention has been described in connection with specific preferred embodiments, it should be understood that the invention as claimed should not be unduly limited to such specific embodiments. Indeed, various modifications of the described modes for carrying out the invention which are obvious to those skilled in molecular biology or related fields are intended to be within the scope of the following claims.

## Claims

1. A method for screening a first repertoire of members comprising polypeptides against a second repertoire of members comprising polypeptides to identify those members of the first repertoire which interact with members of the second repertoire, comprising:
(a) arranging the first repertoire in at least one first series of continuous lines wherein each line of said first series comprises a member of said first repertoire and arranging the second repertoire in at least one second series of continuous lines wherein each line of said second series comprises a member of said second repertoire, wherein the first and second repertoires form at least one array, such that substantially all members of the first repertoire are juxtaposed to substantially all members of the second repertoire; and
(b) detecting an interaction between polypeptides of the first and second repertoires, thereby identifying those members of the first repertoire that interact with members of the second repertoire.

2. The method of claim 1, wherein said first and second repertoires are each present in a series of continuous, non-intersecting lines.

3. A method according to any of claims 1 and 2, wherein the first and second repertoires are arranged on first and second supports respectively.

4. A method according to claim 1 or 2 wherein members of the first and second repertoires are applied to a single support.

5. The method according to any preceding claim , wherein each line of said first and second repertoires are run along channels cut or etched into a solid material such that substantially all the channels containing a member of the first repertoire intersects substantially all the channels containing a member of the second repertoire.

6. The method of any preceding claim wherein the polypeptide chain is a heavy or light chain polypeptide.

7. The method accordng to any of claims 1 to 5, wherein said heavy or light chain polypeptide is a dAb fragment.

8. The method according to claim 6 , wherein said first repertoire comprises V_{H} or V_{L}.

9. The method of claim 7, wherein said second repertoire comprises V_{H} or V_{L}.

10. The method of any of claims 1 to 9, wherein said first repertoire comprises V_{H}, and said second repertoire comprises V_{L}.

11. The method according to any of claims 6 to 10, wherein said step of detecting comprises contacting said at least one array with a target epitope, and detecting binding of the target epitope by juxtaposed members of said first and second repertoires on said array, wherein said binding of the target antigen is indicative of an interaction of members of said first and second repertoire.

12. The method of any of claim 1 to 10, wherein said step of detecting comprises contacting said at least one array with a third repertoire of target antigen members arranged in a series of continuous lines, and detecting binding of target antigen by juxtaposed members of said first and second repertoires at positions on said array, wherein said binding of target antigen is indicative of an interaction of members of said first and second repertoire.

13. The method of claim 12, wherein a plurality of lines of said third repertoire comprise a different target antigen.

14. The method of claim 12, wherein said first and second repertoire are each arranged in a series of continuous, non-intersecting lines.

15. A method for creating a combinatorial library of polypeptides comprising two chains, each member of which library comprises one member of a first repertoire of members comprising a heavy and/or light chain polypeptide and one member of a second repertoire of members comprising a heavy and/or light chain polypeptide, which method comprises the step of arranging the first repertoire of members in a first series of continuous lines, and said second repertoire in a second series of continuous lines, such that substantially all members of the first repertoire are juxtaposed to substantially all members of the second repertoire, thereby generating at the points of juxtaposition, a plurality of combinations of polypeptides comprising two chains, thereby creating a combinatorial library of polypeptides comprising two chains.

16. A method according to claim 15 wherein the combinatorial library produced is screened for interations with more than one target molecule.

17. A method according to claim 12 wherein three chain polypeptide library is created (and optionally screened) using first, second and third repertoires of molecules in three dimensions.

18. A method according to any preceding claim, whereby the pattern of interactiona between the (first, second and optionally third) repertoires is used to identify positive interactions, negative interactions, specific interactions or cross-reactive interactions or is used to construct a phylogenic tree infering the similarity between members of the first reprtoire (using the pattern of interactions with the second and/or, optionally third repertoires) of the second reprtoire (using the pattern of interactions with the first and/or, optionally third repertoires) and/or of the third repertoire (using the pattern of interactions with member of the first and/or second repertoires).

19. The method of claim 18, wherein said first and second repertoires are each arranged in a series of continuous, non-intersecting lines.

20. The method of claim 15, wherein said polypeptides are antibody heavy or light polypeptide chains.

21. The method of claim 15, wherein said polypeptides are dAb fragments.

22. The method of claim 15, wherein said first repertoire comprises V_{H} or V_{L}.

23. The method of claim 15, wherein said second repertoire comprises V_{H} or V_{L}.

24. The method of claim 15, wherein said first repertoire comprises V_{H}, and said second repertoire comprises V_{L}.

25. A method of screening the combinatorial library of two-chain polypeptides of claim 15 for binding to a target molecule, the method comprising the step of detecting an interaction between the two chain polypeptides and the target molecule.

26. The method of claim 25, wherein said step of detecting comprises contacting said combinatorial library of two chain polypeptides with a third repertoire of target antigen members arranged in a series of continuous lines such that a plurality of members of said first, second, and third repertoire is juxtaposed to a plurality of other members of said first, second, and third repertoire, and detecting binding of target antigen by juxtaposed members of said first and second repertoires, wherein said binding of the target antigen is indicative of an interaction of members of said first and second repertoire.

27. The method of claim 26, wherein said first, second and third repertoires are each arranged in a series of continuous, non-intersecting lines.

28. The method of any preceding claim whereby one or more of the first, second and, if present, third repertoires are provided by a plurality of nucleic acid sequences which encode said heavy or light chain polypeptide of said first and second repertoires or said target epitope of said third repertoire and which are expressed to produce their corresponding polypeptides *in situ* in the array.

29. The method according to claim 28, wherein the nucleic acid sequences are provided by expression vectors which encode polypeptide members of the repertoire, and are operatively linked to control sequences sufficient to direct the transcription of the nucleic acid molecules.

30. The method of claim 29, wherein the expression vector is a bacteriophage.

31. The method of claim 29, wherein the expression vector is a plasmid.

32. The method of claim 29, wherein the expression vector is a linear nucleic acid molecule.

33. The method of claim 29, wherein the nucleic acids are contained and expressed within cells.

34. The method according to claim 33, wherein the cells are selected from the group consisting of bacterial cells, lower eukaryotic cells and higher eukaryotic cells.

35. The method of claim 28, wherein the nucleic acid molecules are immobilized in the form of naked or complexed nucleic acid.

36. The method according to any preceding claim wherein the members of at least one repertoire are arrayed using robotic means.

37. A method for screening a first repertoire of molecules against a second repertoire of molecules to identify those members of the first repertoire which do not interact with those members of the second repertoire, comprising:
(a) arranging the first repertoire in at least one first series of continuous lines wherein each line of said first series comprises a member of said first repertoire and arranging the second repertoire in at least one second series of continuous lines wherein each line of said second series comprises a member of said second repertoire, wherein the first and second repertoires form at least one array, such that substantially all members of the first repertoire are juxtaposed to substantially all members of the second repertoire; and
(b) identifying those members of the first and second repertoires that do not interact with one another.

38. A method for screening a first repertoire of molecules against a second repertoire of molecules to identify members of the first and second repertoires whose interactions with one another are dependant on the presence or absence of a third molecule or set of molecules, comprising:
(a) arranging the first repertoire in at least one first series of continuous lines wherein each line of said first series comprises a member of said first repertoire and arranging the second repertoire in at least one second series of continuous lines wherein each line of said second series comprises a member of said second repertoire, wherein the first and second repertoires form at least one array, such that substantially all members of the first repoertoire are juxtaposed to substantially all members of the second repertoire; and
(b) detecting the interactions between members of the first repertoire and the members of the second repertoire in the presence of different concentrations of the third molecule or set of molecules.

39. A method according to claim 38, wherein the third molecule or set of molecules interacts with certain members of the first repertoire, thereby enabling those members of the first repertoire to interact with certain members of the second repertoire.

40. A method according to claim 38, wherein the interactions between the members of the first and second repertoire require the simultaneous binding of these members to the third molecule or set of molecules.

41. A method according to claim 38, wherein the interactions between the members of the first and second repertoire are enhanced by the presence of a third molecule or set of molecules.

42. A method according to claim 38, wherein the interactions between the members of the first and second repertoire are blocked by the presence of a third molecule or set of molecules.

43. A method according to claim 1, wherein fewer dispensing events are required than the number of interactions to be tested.

44. A method for screening two or more repertoires of enzymes which comprise a two or more enzymic reaction to identify those members of the first repertoire and those members of the second repertoire which together create a given product from a given substrate, which method comprises:
(a) arranging the first repertoire in at least one series of continuous lines wherein each line of said first series comprises a member of said first repertoire and arranging the second repertoire in at least one series of second continuous lines wherein each line of said second series comprises a member of said second repertoire, wherein the first and second repertoires form at least one array, such that substantially all members of the first repertoire are juxtaposed to substantially all members of the second repertoire; and
(b) detecting the formation of product at the intersections of the members of the first and the second repertoires.

45. A method for screening different cellular populations against different viral populations to identify those viral populations that infect those cellular populations, which method comprises:
(a) arranging the cellular populations and the viral populations to form at least one array, wherein the viral populations is arranged in at least one first series of continuous lines wherein each line of said viral population comprises a member of said population and arranging the cellular population in at least one second series of continuous lines wherein each line of said second series comprises a member of said cellular population, wherein the viral and cellular populations form at least one array, such that substantially all members of the cellular population are juxtaposed to substantially all members of the viral population; and
(b) detecting those viral populations that infect those cellular populations.

46. A method for screening different cellular fractions against one another to identify those cellular fractions that contain components which interact with components in other cellular fractions, which method comprises:
(a) arranging the cellular fractions in at least two series of continuous lines wherein each line of said first and second series comprises a member of said cellular fraction and such that substantially all the different cellular fractions are juxtaposed to one another.
(b) detecting the interaction/s at the intersections of the different cellular fractions.

47. A method for screening a peptide repertoire against the same peptide repertoire to identify those members of the peptide repertoire that interact with other members of the peptide repertoire, which method comprises:
(a) arranging the members of the peptide repertoire in at least in at least two series of continuous lines wherein each line of said first and second series comprises a member of said peptide repertoire and such that substantially all the different members of the peptide repertoire are juxtaposed to one another
(b) detecting the interaction/s at the intersections of the different members of the peptide repertoire.

48. A method according to claims 46 or 47, which method use the yeast two-hybrid system to identify those members of the repertoires of molecules that interact with one another,

49. A method for creating (and optionally screening) a yeast two hybrid library consisting of substantially all members of a first repertoire of polypeptides paired with substantially all members of a second repertoire of polypeptides, which method comprises:
(a) arranging yeast cells containing a plurality of nucleotide sequences to create at least one array, wherein the nucleotide sequences are arranged in at least one first series of continuous lines wherein each line of said first series comprises yeast cells containing a nucleotide sequence and arranging the second repertoire in at least one second series of continuous lines wherein each line of said second series comprises yeast cells containing a member of said second repertoire, wherein the first and second repertoires form at least one array, such that substantially all members of the first repertoire are juxtaposed to substantially all members of the second repertoire;
(b) allowing the yeast cells containing the members of the first repertoire to mate with the yeast cells containing the members of the second repertoire where the two repertoires intersect; and optionally
(c) expressing the nucleotide sequences to produce the corresponding polypeptides of the first and second repertoires; and optionally
(d) detecting the interaction/s between the polypeptide members of the first and second repertoires.

## Patentansprüche

1. Screeningverfahren für ein erstes Repertoire von Mitgliedern, die Polypeptide gegen ein zweites Repertoire von Mitgliedern umfassen, welche Polypeptide umfassen, um die Mitglieder des ersten Repertoires zu identifizieren, die mit den Mitgliedern des zweiten Repertoires interagieren, umfassend:
(a) Anordnen des ersten Repertoires in mindestens einer Serie kontinuierlicher Reihen, wobei jede Reihe dieser ersten Serie ein Mitglied dieses ersten Repertoires umfaßt, und Anordnen des zweiten Repertoires in mindestens einer zweiten Serie kontinuierlicher Reihen, wobei jede Reihe dieser zweiten Serie ein Mitglied dieses zweiten Repertoires umfaßt, wobei die ersten und zweiten Repertoires mindestens eine Anordnung bilden, in der im wesentlichen alle Mitglieder des ersten Repertoires im wesentlichen neben allen Mitgliedern des zweiten Repertoires vorliegen; und
(b) Nachweisen einer Interaktion zwischen Polypeptiden der ersten und zweiten Repertoires und **dadurch** Identifizieren solcher Mitglieder des ersten Repertoires, die mit Mitgliedern des zweiten Repertoires interagieren.

2. Verfahren nach Anspruch 1, wobei die ersten und zweiten Repertoires jeweils in einer Serie kontinuierlicher sich nicht kreuzender Reihen vorliegen.

3. Verfahren nach irgendeinem der Ansprüche 1 und 2, wobei die ersten und zweiten Repertoires jeweils auf ersten und zweiten Trägern angeordnet sind.

4. Verfahren nach Anspruch 1 oder 2, wobei die Mitglieder der ersten und zweiten Repertoires auf einen einzelnen Träger aufgetragen sind.

5. Verfahren nach irgendeinem vorhergehenden Anspruch, wobei jede Reihe dieser ersten und zweiten Repertoires entlang von Kanälen laufen, die in ein festes Material geschnitten oder geätzt wurden, so daß im wesentlichen alle Kanäle, die ein Mitglied des ersten Repertoires enthalten, im wesentlichen alle Kanäle, die ein Mitglied des zweiten Repertoires enthalten, kreuzen.

6. Verfahren nach irgendeinem vorhergehenden Anspruch, wobei die Polypeptidkette ein Polypeptid der schweren oder leichten Kette ist.

7. Verfahren nach irgendeinem der Ansprüche 1 bis 5, wobei das Polypeptid der schweren oder leichten Kette ein dAb-Fragment ist.

8. Verfahren nach Anspruch 6, wobei das erste Repertoire V_{H} oder V_{L} umfaßt.

9. Verfahren nach Anspruch 7, wobei das zweite Repertoire V_{H} oder V_{L} umfaßt.

10. Verfahren nach irgendeinem der Ansprüche 1 bis 9, wobei das erste Repertoire V_{H} umfaßt und das zweite Repertoire V_{L} umfaßt.

11. Verfahren nach irgendeinem der Ansprüche 6 bis 10, wobei der Schritt des Nachweisens das Inkontaktbringen mindestens dieser einen Anordnung mit einem Zielepitop umfaßt sowie das Nachweisen der Bindung des Zielepitops durch benachbarte Mitglieder der ersten und zweiten Repertoires auf dieser Anordnung, wobei die Bindung des Zielantigens eine Interaktion von Mitgliedern dieser ersten und zweiten Repertoires anzeigt.

12. Verfahren nach irgendeinem der Ansprüche 1 bis 10, wobei der Schritt des Nachweisens das Inkontaktbringen mindestens einer Anordnung mit einem dritten Repertoire aus Zielantigenmitgliedern umfaßt, die in einer Serie kontinuierlicher Reihen angeordnet sind, und das Nachweisen der Bindung eines Zielantigens durch benachbarte Mitglieder dieser ersten und zweiten Repertoires an Positionen dieser Anordnung, wobei die Bindung des Zielantigens eine Interaktion der Mitglieder dieser ersten und zweiten Repertoires anzeigt.

13. Verfahren nach Anspruch 12, wobei eine Vielzahl von Reihen dieses dritten Repertoires ein unterschiedliches Zielantigen umfassen.

14. Verfahren nach Anspruch 12, wobei dieses erste und zweite Repertoire jeweils in einer Serie kontinuierlicher sich nicht überschneidender Reihen angeordnet ist.

15. Verfahren zum Erstellen einer kombinatorischen Bibliothek aus Polypeptiden, die zwei Ketten umfassen, wobei jedes Mitglied dieser Bibliothek ein Mitglied eines ersten Repertoires aus Mitgliedern umfaßt, die ein Polypeptid einer schweren und/oder leichten Kette umfassen, und ein Mitglied eines zweiten Repertoires aus Mitgliedern, die ein Polypeptid einer schweren und/oder leichten Kette umfassen, wobei das Verfahren den Schritt des Anordnens des ersten Repertoires von Mitgliedern in einer ersten Serie kontinuierlicher Reihen und das zweite Repertoire in einer zweiten Serie kontinuierlicher Reihen umfaßt, so daß im wesentlichen alle Mitglieder des ersten Repertoires im wesentlichen neben allen Mitgliedern des zweiten Repertoires vorliegen, wodurch an den nebeneinander liegenden Punkten eine Vielzahl an Kombinationen aus Polypeptiden erzeugt werden, die zwei Ketten umfassen, wodurch eine kombinatorische Bibliothek von Polypeptiden, die zwei Ketten umfassen, generiert wird.

16. Verfahren nach Anspruch 15, wobei die generierte kombinatorische Bibliothek, auf Interaktion mit mehr als einem Zielmolekül durchmustert wird.

17. Verfahren nach Anspruch 12, wobei eine Dreiketten-Polypeptid-Bibliothek generiert (und gegebenenfalls durchmustert) wird, indem erste, zweite und dritte Repertoires von Molekülen in drei Dimensionen verwendet werden.

18. Verfahren nach irgendeinem vorhergehenden Anspruch, wobei das Muster der Interaktionen zwischen den (ersten, zweiten und gegebenenfalls dritten) Repertoires verwendet wird, um positive Interaktionen, negative Interaktionen, spezifische Interaktionen oder kreuzreagierende Interaktionen zu identifizieren, oder verwendet wird, um einen phylogenetischen Baum zu erstellen, der die Ähnlichkeit zwischen Mitgliedern des ersten Repertoires (unter Verwendung des Interaktionsmusters mit dem zweiten und/oder gegebenenfalls dritten Repertoire), des zweiten Repertoires (unter Verwendung des Interaktionsmusters mit dem ersten und/oder gegebenenfalls dritten Repertoire) und/oder des dritten Repertoires (unter Verwendung des Interaktionsmusters mit Mitgliedern des ersten und/oder zweiten Repertoires) ableitet.

19. Verfahren nach Anspruch 18, wobei das erste und zweite Repertoire jeweils in einer Serie kontinuierlicher, sich nicht überschneidender Reihen angeordnet sind.

20. Verfahren nach Anspruch 15, wobei die Polypeptide die schweren oder leichten Polypeptidketten eines Antikörpers sind.

21. Verfahren nach Anspruch 15, wobei diese Polypeptide dAb-Fragmente sind.

22. Verfahren nach Anspruch 15, wobei das erste Repertoire V_{H} oder V_{L} umfaßt.

23. Verfahren nach Anspruch 15, wobei das zweite Repertoire V_{H} oder V_{L} umfaßt.

24. Verfahren nach Anspruch 15, wobei das erste Repertoire V_{H} umfaßt und das zweite Repertoire V_{L} umfaßt.

25. Verfahren zum Durchmustern der kombinatorischen Bibliothek aus zweikettigen Polypeptiden nach Anspruch 15 zur Bindung eines Zielmoleküls, wobei das Verfahren den Schritt des Nachweisens einer Interaktion zwischen den zweikettigen Polypeptiden und dem Zielmolekül umfaßt.

26. Verfahren nach Anspruch 25, wobei der Schritt des Nachweisens das Inkontaktbringen dieser kombinatorischen Bibliothek aus zweikettigen Polypeptiden mit einem dritten Repertoire aus Zielantigenmitgliedern umfaßt, die in einer Serie kontinuierlicher Reihen angeordnet sind, so daß eine Vielzahl von Mitgliedern dieser ersten, zweiten und dritten Repertoires einer Vielzahl von anderen Mitgliedern dieser ersten, zweiten und dritten Repertoires benachbart ist, und Nachweisen der Bindung eines Zielantigens durch benachbarte Mitglieder dieser ersten und zweiten Repertoires, wobei die Bindung des Zielantigens eine Interaktion von Mitgliedern dieser ersten und zweiten Repertoires anzeigt.

27. Verfahren nach Anspruch 26, wobei die ersten, zweiten und dritten Repertoires jeweils in einer Serie kontinuierlicher sich nicht überschneidender Reihen angeordnet sind.

28. Verfahren nach irgendeinem vorhergehenden Anspruch, wobei ein oder mehrere des ersten, zweiten und, falls vorhanden, dritten Repertoires durch eine Vielzahl an Nukleinsäuresequenzen bereitgestellt werden, die die Polypeptide der schweren oder leichten Kette der ersten und zweiten Repertoires oder des Zielepitops des dritten Repertoires codieren, und die exprimiert werden, um ihre entsprechenden Polypeptide in situ in der Anordnung zu produzieren.

29. Verfahren nach Anspruch 28, wobei die Nukleinsäuresequenzen durch Expressionsvektoren bereitgestellt werden, die Polypeptidmitglieder des Repertoires codieren und operativ an Kontrollsequenzen gebunden sind, die ausreichen, um die Transkription der Nukleinsäuremoleküle zu steuern.

30. Verfahren nach Anspruch 29, wobei der Expressionsvektor ein Bacteriophage ist.

31. Verfahren nach Anspruch 29, wobei der Expressionsvektor ein Plasmid ist.

32. Verfahren nach Anspruch 29, wobei der Expressionsvektor ein lineares Nukleinsäuremolekül ist.

33. Verfahren nach Anspruch 29, wobei die Nukleinsäuren in den Zellen enthalten und exprimiert werden.

34. Verfahren nach Anspruch 33, wobei die Zellen aus der Gruppe ausgewählt sind, die aus bakteriellen Zellen, niederen eukaryontischen Zellen und höheren eukaryontischen Zellen besteht.

35. Verfahren nach Anspruch 28, wobei die Nukleinsäuremoleküle in Form nackter oder komplexierter Nukleinsäure immobilisiert sind.

36. Verfahren nach irgendeinem vorhergehenden Anspruch, wobei die Mitglieder mindestens eines Repertoires unter Verwendung von Robotervorrichtungen angeordnet werden.

37. Screeningverfahren eines ersten Repertoires von Molekülen gegen ein zweites Repertoire von Molekülen, um solche Mitglieder des ersten Repertoires zu identifizieren, die nicht mit den Mitgliedern des zweiten Repertoires interagieren, umfassend:
(a) Anordnen des ersten Repertoires in mindestens einer Serie kontinuierlicher Reihen, wobei jede Reihe dieser ersten Serie ein Mitglied dieses ersten Repertoires umfaßt, und Anordnen des zweiten Repertoires in mindestens einer zweiten Serie kontinuierlicher Reihen, wobei jede Reihe dieser zweiten Serie ein Mitglied dieses zweiten Repertoires umfaßt, wobei die ersten und zweiten Repertoires mindestens eine Anordnung bilden, in der im wesentlichen alle Mitglieder des ersten Repertoires im wesentlichen neben allen Mitgliedern des zweiten Repertoires vorliegen; und
(b) Identifizieren solcher Mitglieder der ersten und zweiten Repertoires, die nicht miteinander interagieren.

38. Screeningverfahren eines ersten Repertoires von Molekülen gegen ein zweites Repertoire von Molekülen, um Mitglieder der ersten und zweiten Repertoires zu identifizieren, deren Interaktionen miteinander von der Gegenwart oder Abwesenheit eines dritten Moleküls oder Molekülsatzes abhängen, umfassend:
(a) Anordnen des ersten Repertoires in mindestens einer Serie kontinuierlicher Reihen, wobei jede Reihe dieser ersten Serie ein Mitglied dieses ersten Repertoires umfaßt, und Anordnen des zweiten Repertoires in mindestens einer zweiten Serie kontinuierlicher Reihen, wobei jede Reihe dieser zweiten Serie ein Mitglied dieses zweiten Repertoires umfaßt, wobei die ersten und zweiten Repertoires mindestens eine Anordnung bilden, in der im wesentlichen alle Mitglieder des ersten Repertoires im wesentlichen neben allen Mitgliedern des zweiten Repertoires vorliegen; und
(b) Nachweisen der Interaktion zwischen Mitgliedern des ersten Repertoires und den Mitgliedern des zweiten Repertoires in Gegenwart verschiedener Konzentrationen des dritten Moleküls oder eines Molekülsatzes.

39. Verfahren nach Anspruch 38, wobei das dritte Molekül oder der Molekülsatz mit bestimmten Mitgliedern des ersten Repertoires interagieren, wodurch diesen Mitgliedern des ersten Repertoires ermöglicht wird, mit bestimmten Mitgliedern des zweiten Repertoires zu interagieren.

40. Verfahren nach Anspruch 38, wobei die Interaktionen zwischen den Mitgliedern der ersten und zweiten Repertoires die gleichzeitige Bindung dieser Mitglieder an das dritte Molekül oder den Molekülsatz benötigen.

41. Verfahren nach Anspruch 38, wobei die Interaktionen zwischen den Mitgliedern der ersten und zweiten Repertoires durch das Vorhandensein eines dritten Moleküls oder Molekülsatzes verstärkt werden.

42. Verfahren nach Anspruch 38, wobei die Interaktionen zwischen den Mitgliedern der ersten und zweiten Repertoires in Gegenwart eines dritten Moleküls oder Molekülsatzes blockiert werden.

43. Verfahren nach Anspruch 1, wobei weniger Auftragungsvorgänge benötigt werden als die Anzahl an Interaktionen, die getestet werden.

44. Screeningverfahren von zwei oder mehr Repertoires von Enzymen, die eine zweite oder mehr enzymatische Reaktionen umfassen, um solche Mitglieder des ersten Repertoires und solche Mitglieder des zweiten Repertoires zu identifizieren, die zusammen ein bestimmtes Produkt aus einem bestimmten Substrat erzeugen, wobei das Verfahren umfaßt:
(a) Anordnen des ersten Repertoires in mindestens einer Serie kontinuierlicher Reihen, wobei jede Reihe dieser ersten Serie ein Mitglied dieses ersten Repertoires umfaßt, und Anordnen des zweiten Repertoires in mindestens einer zweiten Serie kontinuierlicher Reihen, wobei jede Reihe dieser zweiten Serie ein Mitglied dieses zweiten Repertoires umfaßt, wobei die ersten und zweiten Repertoires mindestens eine Anordnung bilden, in der im wesentlichen alle Mitglieder des ersten Repertoires im wesentlichen neben allen Mitgliedern des zweiten Repertoires vorliegen; und
(b) Nachweisen der Bildung eines Produkts an den Schnittstellen der Mitglieder der ersten und zweiten Repertoires.

45. Screeningverfahren verschiedener zellulärer Populationen gegenüber verschiedenen viralen Populationen, um solche viralen Populationen zu identifizieren, die die zellulären Populationen infizieren, wobei das Verfahren umfaßt:
(a) Anordnen der zellulären Populationen und der viralen Populationen, um mindestens eine Anordnung zu bilden, wobei die viralen Populationen in mindestens einer ersten Serie kontinuierlicher Reihen angeordnet sind, wobei jede Reihe dieser viralen Populationen ein Mitglied dieser Population umfaßt, und Anordnen der zellulären Population in mindestens einer zweiten Serie kontinuierlicher Reihen, wobei jede Reihe dieser zweiten Serie ein Mitglied dieser zellulären Population umfaßt, wobei die viralen und zellulären Populationen mindestens eine Anordnung bilden, damit im wesentlichen alle Mitglieder der zellulären Population im wesentlichen allen Mitgliedern der viralen Population benachbart sind; und
(b) Nachweisen solcher viraler Populationen, die die zellulären Populationen infizieren.

46. Screeningverfahren verschiedener zellulärer Fraktionen gegen einander, um solche zellulären Fraktionen zu identifizieren, die Bestandteile enthalten, welche mit Bestandteilen in anderen zellulären Fraktionen interagieren, wobei das Verfahren umfaßt:
(a) Anordnen der zellulären Fraktionen in mindestens zwei Serien kontinuierlicher Reihen, wobei jede Reihe dieser ersten und zweiten Serien ein Mitglied dieser zellulären Fraktion umfaßt damit im wesentlichen alle verschiedenen zellulären Fraktionen einander benachbart sind;
(b) Nachweisen der Interaktion(en) an den Schnittstellen der verschiedenen zellulären Fraktionen.

47. Screeningverfahren eines Peptidrepertoires gegen das gleiche Peptidrepertoire, um solche Mitglieder des Peptidrepertoires zu identifizieren, die mit anderen Mitgliedern des Peptidrepertoires interagieren, wobei das Verfahren umfaßt:
(a) Anordnen der Mitglieder des Peptidrepertoires in mindestens zwei Serien kontinuierlicher Reihen, wobei jede Reihe dieser ersten und zweiten Serien ein Mitglied dieses Peptidrepertoires umfasst, damit im wesentlichen alle die verschiedenen Mitglieder des Peptidrepertoires einander benachbart sind;
(b) Nachweisen der Interaktion(en) an den Schnittstellen der verschiedenen Mitglieder des Peptidrepertoires.

48. Verfahren nach den Ansprüchen 46 oder 47, wobei das Verfahren das Hefe-Zwei-Hybrid-System verwendet, um solche Mitglieder der Repertoires an Molekülen zu identifizieren, die miteinander interagieren.

49. Verfahren zum Erzeugen (und gegebenenfalls Durchmustern) einer Hefe-Zwei-Hybrid-Bibliothek, die im wesentlichen aus allen Mitgliedern eines Repertoires an Polypeptiden besteht, die mit im wesentlichen allen Mitgliedern eines zweiten Repertoires an Polypeptiden gepaart werden, wobei das Verfahren umfaßt:
(a) Anordnen von Hefezellen, die eine Vielzahl von Nukleotidsequenzen enthalten, um mindestens eine Anordnung zu bilden, wobei die Nukleotidsequenzen in mindestens einer ersten Serie kontinuierlicher Reihen angeordnet sind, wobei jede Reihe dieser ersten Serie Hefezellen umfaßt, die eine Nukleotidsequenz enthalten, und Anordnen des zweiten Repertoires in mindestens einer zweiten Serie kontinuierlicher Reihen, wobei jede Reihe dieser zweiten Serie Hefezellen umfaßt, die ein Mitglied dieses zwei Repertoires enthalten, wobei die ersten und zweiten Repertoires mindestens eine Anordnung bilden, so daß im wesentlichen alle Mitglieder des ersten Repertoires im wesentlichen allen Mitgliedern des zweiten Repertoires benachbart sind;
(b) Ermöglichen, dass Hefezellen, die die Mitglieder des ersten Repertoires enthalten, sich mit den Hefezellen zu kreuzen, die Mitglieder des zweiten Repertoires enthalten, wo die zwei Repertoires einander kreuzen; und gegebenenfalls
(c) Exprimieren der Nukleotidsequenzen, um die entsprechenden Polypeptide der ersten und zweiten Repertoires zu produzieren; und gegebenenfalls
(d) Nachweisen der Interaktion(en) zwischen Polypeptidmitgliedern der ersten und zweiten Repertoires.

## Revendications

1. Procédé de criblage d'un premier répertoire immunologique de membres comprenant des polypeptides sur un deuxième répertoire immunologique de membres comprenant des polypeptides pour identifier les membres du premier répertoire immunologique qui interagissent avec les membres du deuxième répertoire immunologique, comprenant :
(a) l'agencement du premier répertoire immunologique dans au moins une première série de lignes continues dans laquelle chaque ligne de ladite première série comprend un membre dudit premier répertoire immunologique et l'agencement du deuxième répertoire immunologique dans au moins une deuxième série de lignes continues dans laquelle chaque ligne de ladite deuxième série comprend un membre dudit deuxième répertoire immunologique, dans lequel les premier et deuxième répertoires immunologiques forment au moins une matrice, de telle sorte que sensiblement tous les membres du premier répertoire immunologique soient juxtaposés sensiblement à tous les membres du deuxième répertoire immunologique ; et
(b) la détection d'une interaction entre les polypeptides des premier et deuxième répertoires immunologiques, identifiant ainsi les membres du premier répertoire immunologique qui interagissent avec les membres du deuxième répertoire immunologique.

2. Procédé selon la revendication 1, dans lequel lesdits premier et deuxième répertoires immunologiques sont chacun présents dans une série de lignes continues non entrecroisées.

3. Procédé selon l'une quelconque des revendications 1 et 2, dans lequel les premier et deuxième répertoires immunologiques sont agencés sur des premier et deuxième supports respectivement.

4. Procédé selon la revendication 1 ou 2, dans lequel les membres des premier et deuxième répertoires immunologiques sont appliqués sur un seul support.

5. Procédé selon l'une quelconque des revendications précédentes, dans lequel chaque ligne desdits premier et deuxième répertoires immunologiques s'étend le long de canaux tracés ou gravés dans un matériau solide de telle sorte que sensiblement tous les canaux contenant un membre du premier répertoire immunologique s'entrecroisent sensiblement avec tous les canaux contenant un membre du deuxième répertoire immunologique .

6. Procédé selon l'une quelconque des revendications précédentes, dans lequel la chaîne polypeptidique est un polypeptide à chaîne lourde ou légère.

7. Procédé selon l'une quelconque des revendications 1 à 5, dans lequel ledit polypeptide à chaîne lourde ou légère est un fragment dAb.

8. Procédé selon la revendication 6, dans lequel ledit premier répertoire immunologique comprend V_{H} ou V_{L}.

9. Procédé selon la revendication 7, dans lequel ledit deuxième répertoire immunologique comprend V_{H} ou V_{L}.

10. Procédé selon l'une quelconque des revendications 1 à 9, dans lequel ledit premier répertoire immunologique comprend V_{H}, et ledit deuxième répertoire immunologique comprend V_{L}.

11. Procédé selon l'une quelconque des revendications 6 à 10, dans lequel ladite étape de détection comprend la mise en contact de ladite au moins une matrice avec un épitope cible, et la détection de la liaison de l'épitope cible par des membres juxtaposés desdits premier et deuxième répertoires immunologiques sur ladite matrice, dans lequel ladite liaison de l'antigène cible constitue une indication d'une interaction des membres desdits premier et deuxième répertoires immunologiques.

12. Procédé selon l'une quelconque des revendications 1 à 10, dans lequel ladite étape de détection comprend la mise en contact de ladite au moins une matrice avec un troisième répertoire immunologique de membres antigènes cibles agencés dans une série de lignes continues, et la détection de la liaison de l'antigène cible par des membres juxtaposés desdits premier et deuxième répertoires immunologiques au niveau de positions sur ladite matrice, dans lequel ladite liaison de l'antigène cible constitue une indication d'une interaction des membres desdits premier et deuxième répertoires immunologiques.

13. Procédé selon la revendication 12, dans lequel une pluralité de lignes dudit troisième répertoire immunologique comprennent un antigène cible différent.

14. Procédé selon la revendication 12, dans lequel lesdits premier et deuxième répertoires immunologiques sont chacun agencés dans une série de lignes continues non entrecroisées.

15. Procédé de génération d'une bibliothèque combinatoire de polypeptides comprenant deux chaînes, chaque membre de la bibliothèque comprenant un membre d'un premier répertoire immunologique de membres comprenant un polypeptide à chaîne lourde et/ou légère et un membre d'un deuxième répertoire immunologique de membres comprenant un polypeptide à chaîne lourde et/ou légère, lequel procédé comprend l'étape d'agencement du premier répertoire immunologique de membres dans une première série de lignes continues, et dudit deuxième répertoire immunologique dans une deuxième série de lignes continues, de telle sorte que sensiblement tous les membres du premier répertoire immunologique soient juxtaposés sensiblement à tous les membres du deuxième répertoire immunologique, générant ainsi aux points de juxtaposition, une pluralité de combinaisons de polypeptides comprenant deux chaînes, générant ainsi une bibliothèque combinatoire de polypeptides comprenant deux chaînes.

16. Procédé selon la revendication 15, dans lequel la bibliothèque combinatoire produite est criblée en vue de détecter les interactions avec plusieurs molécules cibles.

17. Procédé selon la revendication 12, dans lequel une bibliothèque de polypeptides à trois chaînes est générée (et éventuellement criblée) en utilisant les premier, deuxième et troisième répertoires immunologiques de molécules en trois dimensions.

18. Procédé selon l'une quelconque des revendications précédentes, moyennant quoi le modèle d'interactions entre les (premier, deuxième et éventuellement troisième) répertoires immunologiques est utilisé pour identifier les interactions positives, les interactions négatives, les interactions spécifiques ou les interactions de réaction croisée ou est utilisé pour construire un arbre phylétique pour déduire la similarité entre les membres du premier répertoire immunologique (en utilisant le modèle d'interactions avec le deuxième et/ou, éventuellement, le troisième répertoire(s) immunologique(s)), du deuxième répertoire immunologique (en utilisant le modèle d'interactions avec le premier et/ou, éventuellement, le troisième répertoire (s) immunologique (s)), et/ou du troisième répertoire immunologique (en utilisant le modèle d'interactions avec les membres du premier et/ou du deuxième répertoire(s) immunologique(s)).

19. Procédé selon la revendication 18, dans lequel lesdits premier et deuxième répertoires immunologiques sont chacun agencés dans une série de lignes continues non entrecroisées.

20. Procédé selon la revendication 15, dans lequel lesdits polypeptides sont des chaînes polypeptidiques lourdes ou légères d'anticorps.

21. Procédé selon la revendication 15, dans lequel lesdits polypeptides sont des fragments dAb.

22. Procédé selon la revendication 15, dans lequel ledit premier répertoire immunologique comprend V_{H} ou V_{L}.

23. Procédé selon la revendication 15, dans lequel ledit deuxième répertoire immunologique comprend V_{H} ou V_{L}.

24. Procédé selon la revendication 15, dans lequel ledit premier répertoire immunologique comprend V_{H} et ledit deuxième répertoire immunologique comprend V_{L}.

25. Procédé de criblage de la bibliothèque combinatoire de polypeptides à deux chaînes selon la revendication 15, pour la liaison à une molécule cible, le procédé comprenant l'étape de détection d'une interaction entre les polypeptides à deux chaînes et la molécule cible.

26. Procédé selon la revendication 25, dans lequel ladite étape de détection comprend la mise en contact de ladite bibliothèque combinatoire de polypeptides à deux chaînes avec un troisième répertoire immunologique de membres antigènes cibles agencés dans une série de lignes continues de telle sorte qu'une pluralité de membres desdits premier, deuxième, et troisième répertoires immunologiques soient juxtaposés à une pluralité d'autres membres desdits premier, deuxième, et troisième répertoires immunologiques, et la détection de la liaison de l'antigène cible par des membres juxtaposés desdits premier et deuxième répertoires immunologiques, dans lequel ladite liaison de l'antigène cible constitue une indication d'une interaction des membres desdits premier et deuxième répertoires immunologiques.

27. Procédé selon la revendication 26, dans lequel lesdits premier, deuxième, et troisième répertoires immunologiques sont chacun agencés dans une série de lignes continues non entrecroisées.

28. Procédé selon l'une quelconque des revendications précédentes, moyennant quoi un ou plusieurs des premier, deuxième et, s'il est présent, troisième répertoires immunologiques sont fournis par une pluralité de séquences d'acides nucléiques qui codent pour ledit polypeptide à chaîne lourde ou légère desdits premier et deuxième répertoires immunologiques ou ledit épitope cible dudit troisième répertoire immunologique et qui sont exprimés pour produire leurs polypeptides correspondants *in situ* dans la matrice.

29. Procédé selon la revendication 28, dans lequel les séquences d'acides nucléiques sont fournies par des vecteurs d'expression qui codent pour des membres polypeptidiques du répertoire immunologique, et sont fonctionnellement liées aux séquences de contrôle suffisamment pour diriger la transcription des molécules d'acide nucléique.

30. Procédé selon la revendication 29, dans lequel le vecteur d'expression est un bactériophage.

31. Procédé selon la revendication 29, dans lequel le vecteur d'expression est un plasmide.

32. Procédé selon la revendication 29, dans lequel le vecteur d'expression est une molécule d'acide nucléique linéaire.

33. Procédé selon la revendication 29, dans lequel les acides nucléiques sont contenus et exprimés au sein de cellules.

34. Procédé selon la revendication 33, dans lequel les cellules sont choisies dans le groupe consistant en des cellules bactériennes, des cellules eucaryotes inférieures et des cellules eucaryotes supérieures.

35. Procédé selon la revendication 28, dans lequel les molécules d'acide nucléique sont immobilisées dans la forme d'acide nucléique nu ou complexé.

36. Procédé selon l'une quelconque des revendications précédentes, dans lequel les membres d'au moins un répertoire immunologique sont déployés en utilisant un procédé robotisé.

37. Procédé de dépistage d'un premier répertoire immunologique de molécules sur un deuxième répertoire immunologique de molécules pour identifier les membres du premier répertoire immunologique qui n'interagissent pas avec les membres du deuxième répertoire immunologique, comprenant :
(a) l'agencement du premier répertoire immunologique dans au moins une première série de lignes continues dans laquelle chaque ligne de ladite première série comprend un membre dudit premier répertoire immunologique et l'agencement du deuxième répertoire immunologique dans au moins une deuxième série de lignes continues dans laquelle chaque ligne de ladite deuxième série comprend un membre dudit deuxième répertoire immunologique, dans lequel les premier et deuxième répertoires immunologiques forment au moins une matrice, de telle sorte que sensiblement tous les membres du premier répertoire immunologique soient juxtaposés sensiblement à tous les membres du deuxième répertoire immunologique ; et
(b) l'identification des membres des premier et deuxième répertoires immunologiques qui n'interagissent pas les uns avec les autres.

38. Procédé de criblage d'un premier répertoire immunologique de molécules sur un deuxième répertoire immunologique de molécules pour identifier les membres du premier et du deuxième répertoires immunologiques dont les interactions les uns avec les autres dépendent de la présence ou de l'absence d'une troisième molécule ou d'un troisième ensemble de molécules, comprenant :
(a) l'agencement du premier répertoire immunologique dans au moins une première série de lignes continues dans laquelle chaque ligne de ladite première série comprend un membre dudit premier répertoire immunologique et l'agencement du deuxième répertoire immunologique dans au moins une deuxième série de lignes continues dans laquelle chaque ligne de ladite deuxième série comprend un membre dudit deuxième répertoire immunologique, dans lequel les premier et deuxième répertoires immunologiques forment au moins une matrice, de telle sorte que sensiblement tous les membres du premier répertoire immunologique soient juxtaposés sensiblement à tous les membres du deuxième répertoire immunologique ; et
(b) la détection des interactions entre les membres du premier répertoire immunologique et les membres du deuxième répertoire immunologique en présence de différentes concentrations de la troisième molécule ou du troisième ensemble de molécules.

39. Procédé selon la revendication 38, dans lequel la troisième molécule ou le troisième ensemble de molécules interagissent avec certains membres du premier répertoire immunologique, permettant ainsi aux membres du premier répertoire immunologique d'interagir avec certains membres du deuxième répertoire immunologique.

40. Procédé selon la revendication 38, dans lequel les interactions entre les membres du premier et du deuxième répertoires immunologiques nécessitent la liaison simultanée de ces membres à la troisième molécule ou au troisième ensemble de molécules.

41. Procédé selon la revendication 38, dans lequel les interactions entre les membres du premier et du deuxième répertoires immunologiques sont amplifiées par la présence d'une troisième molécule ou d'un troisième ensemble de molécules.

42. Procédé selon la revendication 38, dans lequel les interactions entre les membres du premier et du deuxième répertoires immunologiques sont bloquées par la présence d'une troisième molécule ou d'un troisième ensemble de molécules.

43. Procédé selon la revendication 1, dans lequel il est nécessaire d'avoir moins d'événements d'administration que le nombre d'interactions à tester.

44. Procédé de criblage de deux ou plusieurs répertoires immunologiques d'enzymes qui comprennent deux ou plusieurs réactions enzymatiques pour identifier les membres du premier répertoire immunologique et les membres du deuxième répertoire immunologique qui génèrent conjointement un produit donné à partir d'un substrat donné, lequel procédé comprend :
(a) l'agencement du premier répertoire immunologique dans au moins une série de lignes continues dans laquelle chaque ligne de ladite première série comprend un membre dudit premier répertoire immunologique et l'agencement du deuxième répertoire immunologique dans au moins une série de deuxièmes lignes continues dans laquelle chaque ligne de ladite deuxième série comprend un membre dudit deuxième répertoire immunologique, dans lequel les premier et deuxième répertoires immunologiques forment au moins une matrice, de telle sorte que sensiblement tous les membres du premier répertoire immunologique soient juxtaposés sensiblement à tous les membres du deuxième répertoire immunologique ; et
(b) la détection de la formation de produit aux intersections des membres du premier et du deuxième répertoires immunologiques.

45. Procédé de criblage de différentes populations cellulaires sur différentes populations virales pour identifier les populations virales qui infectent ces populations cellulaires, lequel procédé comprend:
(a) l'agencement des populations cellulaires et des populations virales pour former au moins une matrice, dans lequel les populations virales sont agencées dans au moins une première série de lignes continues dans laquelle chaque ligne de ladite population virale comprend un membre de ladite population et l'agencement de la population cellulaire dans au moins une deuxième série de lignes continues dans laquelle chaque ligne de ladite deuxième série comprend un membre de ladite population cellulaire, dans lequel les populations virales et cellulaires forment au moins une matrice, de telle sorte que sensiblement tous les membres de la population cellulaire soient juxtaposés sensiblement à tous les membres de la population virale ; et
(b) la détection des populations virales qui infectent ces populations cellulaires.

46. Procédé de criblage de différentes fractions cellulaires les unes sur les autres pour identifier les fractions cellulaires qui contiennent des composants qui interagissent avec des composants dans d'autres fractions cellulaires, lequel procédé comprend :
(a) l'agencement des fractions cellulaires dans au moins deux séries de lignes continues dans lesquelles chaque ligne desdites première et deuxième séries comprend un membre de ladite fraction cellulaire et de telle sorte que sensiblement toutes les différentes fractions cellulaires soient juxtaposés les unes aux autres.
(b) la détection des interactions aux intersections des différentes fractions cellulaires.

47. Procédé de criblage d'un répertoire immunologique peptidique sur le même répertoire immunologique peptidique pour identifier les membres du répertoire immunologique peptidique qui interagissent avec d'autres membres du répertoire immunologique peptidique, lequel procédé comprend :
(a) l'agencement des membres du répertoire immunologique peptidique dans au moins deux séries de lignes continues dans lesquelles chaque ligne desdites première et deuxième séries comprend un membre dudit répertoire immunologique peptidique et de telle sorte que sensiblement tous les différents membres du répertoire immunologique peptidique soient juxtaposés les uns aux autres.
(b) la détection de l'interaction/des interactions aux intersections des différents membres du répertoire immunologique peptidique.

48. Procédé selon la revendication 46 ou 47, lequel procédé utilise le système double hybride de levure pour identifier les membres des répertoires immunologiques de molécules qui interagissent les uns avec les autres.

49. Procédé de génération (et éventuellement de criblage) d'une bibliothèque de doubles hybrides de levure constituée sensiblement de tous les membres d'un premier répertoire immunologique de polypeptides appariés sensiblement à tous les membres d'un deuxième répertoire immunologique de polypeptides, lequel procédé comprend:
(a) l'agencement de cellules de levure contenant une pluralité de séquences nucléotidiques pour générer au moins une matrice, dans lequel les séquences nucléotidiques sont agencées dans au moins une première série de lignes continues dans laquelle chaque ligne de ladite première série comprend des cellules de levure contenant une séquence nucléotidique et l'agencement du deuxième répertoire immunologique dans au moins une deuxième série de lignes continues dans laquelle chaque ligne de ladite deuxième série comprend des cellules de levure contenant un membre dudit deuxième répertoire immunologique, dans lequel les premier et deuxième répertoires immunologiques forment au moins une matrice, de telle sorte que sensiblement tous les membres du premier répertoire immunologique soient juxtaposés sensiblement à tous les membres du deuxième répertoire immunologique ;
(b) le fait de permettre aux cellules de levure contenant les membres du premier répertoire immunologique de s'apparier aux cellules de levure contenant les membres du deuxième répertoire immunologique là où les deux répertoires immunologiques s'entrecroisent ; et éventuellement
(c) l'expression des séquences nucléotidiques pour produire les polypeptides correspondants du premier et du deuxième répertoires immunologiques ; et éventuellement
(d) la détection de l'interaction/des interactions entre les membres polypeptidiques du premier et du deuxième répertoires immunologiques.
